# FASCICULE DE BREVET EUROPEEN

(11) **EP 0 998 501 B1**
(45) Date de publication et mention de la délivrance du brevet: **27.03.2002**
(21) Numéro de dépôt: 98939713.8
(22) Date de dépôt: 20.07.1998
(51) Int. Cl.: C08F 8/00, C08G 81/02, A61K 47/48

(54) **POLYMERES CATIONIQUES, COMPLEXES ASSOCIANT LESDITS POLYMERES CATIONIQUES ET DES SUBSTANCES THERAPEUTIQUEMENT ACTIVES COMPRENANT AU MOINS UNE CHARGE NEGATIVE, NOTAMMENT DES ACIDES NUCLEIQUES, ET LEUR UTILISATION EN THERAPIE GENIQUE**
KATIONISCHE POLYMERE, DIESE KATIONISCHE POLYMERE ENTHALTENDE KOMPLEXE UND WENIGSTENS EINE NEGATIVE LADUNG ENTHALTENDE THERAPEUTISCH WIRKSAME MITTEL, INSBESONDERE NUKLEINSÄUREN UND IHRE VERWENDUNG IN GENTHERAPIE
CATIONIC POLYMERS, COMPLEXES ASSOCIATING SAID CATIONIC POLYMERS WITH THERAPEUTICALLY ACTIVE SUBSTANCES COMPRISING AT LEAST A NEGATIVE CHARGE, IN PARTICULAR NUCLEIC ACIDS, AND THEIR USE IN GENE THERAPY

(30) Priorité: 21.07.1997 FR 9709209; 12.12.1997 FR 9715807
(43) Date de publication de la demande: 10.05.2000
(73) Titulaire: TRANSGENE S.A., 67000 Strasbourg (FR)
(72) Inventeur: KOLBE, Hanno, V., J., F-67204 Achenheim (FR); BOUSSIF, Otmane, F-67000 Strasbourg (FR); DELAIR, Thierry, F-69700 Echalas (FR); VERON, Laurent, F-69005 Lyon (FR)
(74) Mandataire: Warcoin, Jacques
(86) Numéro de dépôt international: FR9801581
(87) Numéro de publication internationale: WO9905183

(56) Documents cités:
- EP-A- 0 154 742
- EP-A- 0 359 996
- EP-A- 0 580 078
- EP-A- 0 580 079
- EP-A- 0 591 807
- WO-A-92/07023
- WO-A-95/03356
- DE-A- 4 227 019
- US-A- 3 325 472
- US-A- 3 440 320
- US-A- 5 260 385

## Description

La présente invention concerne de nouveaux polymères cationiques utilisables pour former des complexes de polymères cationiques et de substances thérapeutiquement actives comprenant au moins une charge négative et les complexes correspondants, utiles notamment pour le transfert d'une substance thérapeutiquement active, notamment un acide nucléique, dans une cellule cible.

Les maladies génétiques s'expliquent en particulier par un disfonctionnement de l'expression de gènes spécifiques ou par l'expression de polypeptides mutés non fonctionnels dans au moins un type cellulaire. La solution thérapeutique qui semble la mieux adaptée à ce type d'affection est de transférer dans des cellules cibles spécifiques extraites puis réintroduites dans le corps humain, ou directement dans les organes affectés, l'information génétique capable de corriger le défaut observé. Il pourra s'agir par exemple du gène codant pour la protéine CFTR dans le cas de la mucoviscidose ou du gène codant pour la dystrophine dans le cas de la myopathie de Duchenne. Dans le cadre de cette approche, également appelée thérapie génique, l'information génétique est introduite soit *in vitro* dans une cellule extraite de l'organe, la cellule modifiée étant ensuite réintroduite dans l'organisme (procédé *ex vivo*), soit directement *in vivo* dans le tissus approprié. De nombreuses publications décrivent également la mise en oeuvre d'un protocole de thérapie génique afin d'obtenir dans les cellules cibles l'expression d'une protéine présentant un intérêt thérapeutique par introduction de l'information génétique correspondante. L'intérêt thérapeutique peut par exemple résider dans la possibilité d'éliminer une tumeur, ou à défaut de retarder sa progression, par le transfert dans les cellules cancéreuses cibles de gènes immunostimulateurs (immunothérapie) susceptibles d'induire ou d'activer une réponse immune à médiation cellulaire à l'égard de la tumeur, ou l'administration de gènes codant pour les cytokines, de gènes cytotoxiques conférant une toxicité aux cellules les exprimant, par exemple le gène *tk* du virus Herpes Simplex de type 1 (HSV-1), ou d'anti-oncogènes, tels que par exemple le gène associé au rétinoblastome ou p53, ou de polynucléotides capables d'inhiber l'activité d'un oncogène, tels que par exemple les molécules antisens ou les ribozymes capables de dégrader les ARN messagers spécifiques des oncogènes.

Au cours des 30 dernières années, plusieurs travaux ont décrit des techniques relatives au transfert de cette information génétique dans des cellules, notamment des cellules de mammifère. Ces différentes techniques peuvent être divisées en deux catégories. La première catégorie concerne les techniques physiques telles que la microinjection, l'électroporation ou le bombardement particulaire qui, bien qu'efficaces, sont largement limitées à des applications *in vitro* et dont la mise en oeuvre est lourde et délicate. La seconde catégorie fait appel à des techniques relatives à la biologie moléculaire et cellulaire pour lesquelles le matériel génétique à transférer est associé à un vecteur de nature biologique ou synthétique qui favorise l'introduction dudit matériel.

Actuellement, les vecteurs les plus efficaces sont des vecteurs viraux, en particulier adénoviraux ou rétroviraux. Les techniques développées reposent sur les propriétés naturelles dont disposent ces virus pour traverser les membranes cellulaires, échapper à la dégradation de leur matériel génétique et faire pénétrer leur génome dans le noyau cellulaire. Ces virus ont déjà fait l'objet de nombreuses études et certains d'entre eux sont d'ores et déjà employés à titre expérimental comme vecteurs de gènes chez l'homme en vue par exemple d'une vaccination, d'une immunothérapie ou d'une thérapie visant à suppléer une déficience génétique. Toutefois, cette approche virale présente certaines limitations, en particulier liées aux risques de dissémination dans l'organisme hôte et dans l'environnement des particules virales infectieuses produites, au risque de mutagénèse artéfactuel par insertion dans la cellule hôte dans le cas des vecteurs rétroviraux, et à l'induction des réponses immunitaire et inflammatoire *in vivo* lors du traitement thérapeutique. C'est pourquoi des systèmes alternatifs, non viraux de transfert de polynucléotides ont également été développés.

On peut citer par exemple la coprécipitation au phosphate de calcium, l'utilisation de lipides cationiques tels que le DOTMA : N-(1-(2,3-dioleyloxyl)propyl)-N,N,N-triméthylammonium (Felgner et al., 1987, PNAS, 84, 7413-7417), le DOGS : dioctadecylamidoglycylspermine (Behr et al., 1989,PNAS, 86, 6982-6986 ou Transfectam™), le DMRIE : 1,2-dimiristyloxypropyl-3-diméthyl-hydroxyéthylammonium et le DORIE : 1,2-diooleyloxypropyl-3-diméthyl-hydroxyéthylammnoium (Felgner et al., 1993, Methods 5, 67-75), le DC-CHOD : 3β[N-(N',N'-diméthylaminoéthane)-carbamoyl]cholestérol (Gao et Huang, 1991, BBRC, 179, 280-285), le DOTAP™ (McLachlan et al., 1995, Gene Therapy, 2,674-622) ou la Lipofectamine™; ou l'utilisation de polymères couplés à des ligands reconnus par un récepteur membranaire (pour une revue voir Cotten et Wagner, 1993, Current Opinion in Biotechnology, 4, 705-710).

Cependant, un des problèmes majeurs rencontrés lorsque l'on souhaite transférer des gènes dans des cellules cibles, réside dans la difficulté de faire pénétrer les acides nucléiques en raison notamment de leur nature polyanionique qui prévient leur passage à travers les membranes cellulaires. L'utilisation de polymères cationiques pouvant s'associer aux acides nucléiques par liaisons électrostatiques permet de résoudre ce problème au moins partiellement. Ainsi le document WO 95/24221 décrit l'utilisation de polymères dendritiques, le document WO 96/02655, l'utilisation de polyéthylène imine, ou de polypropylène imine et les documents US-A-5 595 897 et FR 2 719 316, l'utilisation de conjugués de polylysine.

Le déposant a aujourd'hui identifié de nouveaux complexes comprenant des polymères cationiques possédant des propriétés particulièrement avantageuses pour le transfert dans les cellules de substances thérapeutiquement actives comportant des charges négatives, notamment des acides nucléiques. En outre, les polymères utilisés présentent l'avantage d'être facilement accessibles, notamment par synthèse chimique et peu coûteux:. Ils sont très faiblement toxiques à l'égard des cellules ce qui constitue un avantage considérable dans le domaine de la thérapie génique.

La présente invention concerne tout d'abord un complexe comprenant au moins un polymère cationique de formule I : 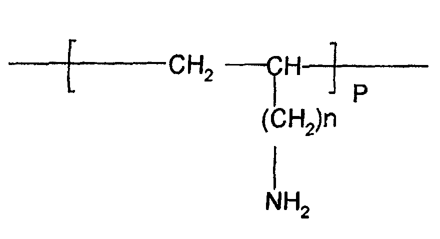 dans laquelle n est un nombre entier variant de 0 à 5 et p est un nombre entier variant de 2 à 20000, plus particulièrement p varie de 10 à 18000, et avantageusement de 200 à 1000
caractérisé en ce que
- au moins 10%, avantageusement de 30 à 80%, préférentiellement 70%, des fonctions NH₂ libres sont substituées par des groupes R hydrophiles, identiques ou différents ;
- ledit polymère cationique peut en outre comprendre au moins un élément de ciblage associé de manière covalente ou non aux fonctions NH2 libres et/ou auxdits groupes R hydrophiles sous réserve que ledit polymère cationique contienne au moins 20%, préférentiellement au moins 30%, de fonctions NH₂ libres, associé à au moins une substance thérpeutiquement active, comportant au moins une charge négative.

Plus particulièrement le polymère cationique, compris dans le complexe suivant l'invention est défini par la formule II suivante :

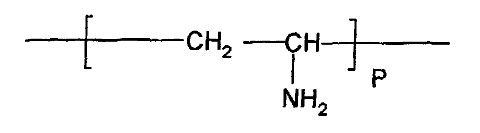

Avantageusement, ledit polymère cationique est défini par la formule III :

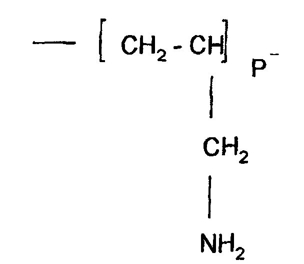

Les polymères de formule II et III présentent les caractéristiques telles que définies préalablement pour le polymère de formule plus générale I.

Selon la présente invention, par « groupe hydrophile » on entend désigner un groupe comportant au moins une fonction hydrophile. Il peut s'agir par exemple d'une fonction hydrophile choisie parmi les fonctions, amine, hydroxyle, amide et ester.

Ces fonctions hydrophiles peuvent être directement associées aux fonctions NH2 libres du polymère par une liaison N-C, ou indirectement par l'intermédiaire d'un bras. Dans ce dernier cas, l'invention concerne par exemple un complexe tel que défini précédemment comprenant au moins un polymère cationique pour lequel R est choisi parmi les groupes :

R' -C = 0,

et ;

-(CH₂)_{n'}-R'

où R' désigne un groupement contenant au moins une fonction hydrophile et n' est un nombre entier variant de 1 à 5.

Selon un mode de réalisation avantageux, le groupe hydrophile R ou R' consiste en un polymère présentant des propriétés hydrophiles, tel que par exemple le polyéthylène glycol (PEG) ou ses dérivés, par exemple un méthoxy-PEG, la polyvinylpyrrolidone, le polymethyloxazoline, le polyethyloxazoline, le polyhydroxypropyl methacrylamide, l'acide polylactique, l'acide polyglycolique et les dérivés de celluloses tels que l'hydroxymethylcellulose ou l'hydroxyethylcellulose. Selon l'invention, le poids moléculaire de tels polymères varie de préférence de 300 à 5000, plus particulièrement de 1000 à 4000, et est avantageusement de 2000. Le polymère préféré pour la mise en oeuvre d'une telle variante de l'invention est le polyéthylène glycol (PEG) et plus particulièrement le PEG 2000.

D'une manière générale, lorsque n et/ou n' sont supérieurs à 5, la solubilité du polymère cationique ainsi formé ou sa capacité à former des interactions stables avec une molécule chargée négativement peuvent être perturbées. Néanmoins, il est à la portée de l'homme du métier d'analyser les propriétés de telles structures et de déterminer par expérimentations les conditions les plus favorables pour l'utilisation de tels polymères, notamment pour la complexation avec des acides nucléiques et pour la transfection de cellules.

Les polymères cationiques utilisables dans la présente invention sont, par exemple, les polyallylamines glycolilées, en particulier un polymère de formule III (n = 1) où : 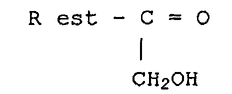 ou encore les polyallylamines éthoxylées, en particulier un polymère de formule IV (n=1) où R est -(CH₂)₂-OH
et environ 50 à environ 80 %, de préférence de 50 à environ 70 %, de fonction NH₂ libres sont substituées par R. Selon un mode de réalisation encore plus particulier p = 592.

Avantageusement, le complexe selon l'invention comprend au moins un polymère cationique tel que défini par les formules I, II, III ou IV et qui comprend en outre au moins un élément de ciblage. De tels éléments de ciblage peuvent permettre de diriger le transfert d'une substance active vers certains types cellulaires ou certains tissus particuliers (cellules tumorales, cellules de l'épithélium pulmonaire, cellule hématopoïétique, cellule musculaire. Ils peuvent également permettre de diriger le transfert d'une substance active vers certains compartiments intracellulaires préférés tels que le noyau, les mitochondries. Il peut en outre s'agir d'éléments facilitant la pénétration à l'intérieur de la cellule ou la lyse des endosomes. De tels éléments de ciblage sont largement décrits dans la littérature. Il peut par exemple s'agir de tout ou partie de lectines, de peptides, notamment le peptide JTS-1 (voir demande de brevet WO 94/40958), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogéniques, de peptides de localisation nucléaire, ou d'une combinaison de tels composés. En particulier, il peut s'agir de résidus galactosyl permettant de cibler le récepteur des asialoglycoprotéines à la surface des cellules hépatiques, de ligands pouvant interagir avec des récepteurs tels que des récepteurs de facteurs de croissance, des récepteur de cytokines, de lectines, de protéines d'adhésion, il peut également s'agir d'un fragment d'anticorps tel que le fragment Fab, d'un peptide fusogénique INF-7 dérivé de la sous unité HA-2 de l'hémagglutinine du virus influenza (Plank et al., 1994, J. Biol. Chem. 269, 12918-12924), d'un signal de localisation nucléaire dérivé de l'antigène T du virus SV40 ou de la protéine EBNA-1 du virus Epstein Barr.

Cet élément de ciblage est lié de manière covalente ou non covalente, au polymère de formule I, ou II, ou III, ou IV. Dans le cas où le polymère cationique est associé à une substance active comportant des charges négatives pour former un complexe selon la présente invention, il est possible que ledit élément de ciblage soit lié à ladite substance active.

De manière avantageuse, un tel complexe est caractérisé en ce que ladite substance thérapeutiquement active est choisie parmi les acides nucléiques et les protéines.

Par "acide nucléique", on entend désigner un fragment d'ADN et/ou d'ARN, double brin ou simple brin, linéaire ou circulaire, naturel isolé ou de synthèse, désignant un enchainement précis de nucléotides, modifiés ou non, permettant de définir un fragment ou une région d'un acide nucléique sans limitation de taille. Selon un mode de réalisation préféré, la substance thérapeutiquement active est un acide nucléique choisi parmi le groupe consistant en un cADN ; un ADN génomique ; un ADN plasmidique ; un ARN messager ; un ARN antisens; un ribozyme; un ARN de transfert; un ARN ribosomique; ou un ADN codant pour de tels ARN ; un polynucléotide libre de tout composé facilitant son introduction dans les cellules; un acide nucléique associé à au moins un polypeptide, notamment un polypeptide d'origine virale, et plus particulièrement d'origine adénovirale ou rétrovirale, ou un polypeptide synthétique; un acide nucléique associé à un ligand; un acide nucléique associé à des amphiphiles, notamment des lipides, cationiques; un acide nucléique associé à des polymères cationiques différents des polymères selon la présente invention ou à des polymères neutres ou anioniques.

Selon une variante de l'invention, ladite substance thérapeutiquement active comprise dans ledit complexe est un acide nucléique qui comprend un gène d'intérêt et des éléments d'expression dudit gène d'intérêt. Un tel gène d'intérêt pourra par exemple coder pour tout ou partie d'un ribozyme ou d'un acide nucléique antisens. Selon un autre mode de réalisation de l'invention, ledit gène d'intérêt code pour tout ou partie d'un polypeptide, notamment d'un polypeptide marqueur (luciférase, β-galactosidase, produit conférant une résistance à un antibiotique ou d'un polypeptide présentant une activité thérapeutique ou prophylactique (polypeptide thérapeutiquement actif), et plus particulièrement une activité immunogène de type cellulaire ou humorale. Le terme polypeptide s'entend sans restriction quant à sa taille ou son degré de glycosylation. On peut également citer à titre d'exemples de gènes d'intérêt, les gènes codant pour un enzyme, un inhibiteur d'enzyme, une hormone, une cytokine, un récepteur membranaire, un polypeptide structural, un polypeptide formant un canal membranaire, un polypeptide de transport, une molécule d'adhésion, un ligand, un facteur de régulation de la transcription, de la traduction, de la réplication, de la stabilisation des transcripts, un facteur de coagulation, un polypeptide à effet antitumoral, un polypeptide capable de ralentir le developpement d'une infection bactérienne, virale ou parasitaire, une toxine,ou un anticorps, tels que par exemple le gène codant pour la protéine CFTR, la dystrophine, le facteur VIII ou IX, E6/E7 de HPV, MUC1, BRCA1, l'interféron β, l'interféron γ, l'interleukine (IL)2, l'IL-4, l'IL-6, l'IL-7, l'IL-12, le facteur nécrosant de tumeur (TNF) de type alpha, le GM-CSF (Granulocyte Macrophage Colony Stimulating Factor), le gène tk du virus Herpes Simplex de type 1 (HSV-1), le gène associé au rétinoblastome ou p53 ou tout ou partie d'immunoglobulines, telles que les fragments F(ab)₂, Fab', Fab ou les anti-idiotypes (US 4,699,880). L'utilisation d'autres gènes peut être envisagée.

Dans le cas où l'acide nucléique comprend tout ou partie d'un gène d'intérêt codant pour tout ou partie d'un polypeptide, il convient de préciser que ledit acide nucléique comporte en outre les éléments nécessaires afin d'assurer l'expression dudit ADN après transfert dans une cellule cible, notamment des séquences promotrices et/ou des séquences de régulation efficaces dans ladite cellule, et éventuellement les séquences requises pour permettre l'excrétion ou l'expression à la surface des cellules cibles dudit polypeptide. A titre d'exemple, on mentionnera les promoteurs tels que les promoteurs des virus RSV (Rous Sarcoma Virus), MPSV, SV40 (Simian virus), CMV (Cytomegalovirus) ou du virus de la vaccine, les promoteurs du gène codant pour la créatine kinase musculaire, pour l'actine, pour le surfactant pulmonaire. Il est en outre possible de choisir une séquence promotrice spécifique d'un type cellulaire donné, ou activable dans des conditions définies. La littérature procure un grand nombre d'informations relatives à de celles séquences promotrices. Par ailleurs, ledit acide nucléique peut comprendre au moins deux séquences, identiques ou différences, présentant une activité de promoteur transcriptionnel et/ou au moins deux séquences codantes d'ADN, identiques ou différences, situées l'une par rapport à l'autre de manière contigué, éloignée, dans le même sens ou en sens inverse, pour autant que la fonction de promoteur transcriptionnel ou la transcription desdites séquences ne soit pas affectée. De même, dans ce type de construction d'acide nucléique, il est possible d'introduire des séquences nucléiques "neutres" ou introns qui ne nuient pas la transcription et sont épissées avant l'étape de traduction. De telles séquences et leurs utilisations sont décrites dans la littérature (WO 94/29471). Ledit acide nucléique pourra également renfermer des séquences requises pour le transport intracellulaire, pour la réplication et/ou pour l'intégration, pour la sécrétion, pour la transcription ou la traduction. De telles séquences sont bien connues de l'homme de l'art. Par ailleurs, les acides nucléiques utilisables selon la présente invention peuvent également être des acides nucléiques modifiés de sorte qu'il ne leur est pas possible de s'intégrer dans le génôme de la cellule cible ou des acides nucléiques stabilisés à l'aide d'agents, tels que par exemple la spermine, qui en tant que tels n'ont pas d'effet sur l'efficacité de la transfection.

Avantageusement, on choisira un rapport spécifique entre le nombre de charges positives dudit polymère cationique et le nombre de charges négatives de ladite substance thérapeutiquement active. On choisira de préférence des quantités des différentes charges de manière à ce que le rapport entre les charges positives du polymère cationique et les charges négatives de la substance active soit compris entre 1 et 30, notamment entre 1.5 et 10, et de préférence entre 2.5 et 5. Le calcul pour arriver à un tel rapport prendra en considération les charges négatives portées par la substance active et on ajustera la quantité de polymère cationique nécessaire pour satisfaire au rapport indiqué ci-dessus. Lorsque le polymère contient également un ou plusieurs éléments de ciblage, les quantités et les concentrations des éléments de ciblage sont ajustées en fonction de leur masse molaire respective et de leur nombre de charges positives et/ou négatives.

L'invention concerne également un procédé de préparation des complexes polymère cationique/ substance thérapeutiquement active selon l'invention, caractérisé en ce que l'on met en présence un ou plusieurs polymères selon l'invention avec une ou plusieurs substances thérapeutiquement actives comportant au moins une charge négative et en ce que l'on récupère ledit complexe. La récupération dudit complexe peut être assortie d'une phase de purification. Plusieurs techniques de purifications sont envisageables telles que celles reposant sur l'utilisation d'un gradient de densité ou d'une colonne d'affinité, spécifique ou non spécifique. Ces techniques de purification sont bien connues de l'homme du métier qui possède les compétences suffisantes à leur mise en oeuvre.

L'invention porte aussi sur un procédé pour transférer une substance thérapeutiquement active en particulier un acide nucléique dans une cellule cible in vitro, ex vivo ou in vivo caractérisé en ce que l'on met en contact au moins un complexe selon l'invention avec des cellules cibles. Selon un mode de réalisation du procédé selon l'invention, on met en contact des cellules cultivées sur un milieu approprié avec une suspension comprenant au moins un complexe polymère cationique/substance comportant des charges négatives tel que décrit dans la présence invention. Après un certain temps d'incubation, les cellules sont lavées et récupérées. La vérification de la transfection peut être effectuée par tout moyen approprié, et notamment par mesure de l'expression du gène porté par l'acide nucléique formant complexe avec le polymère cationique ou par mesure de la concentration du polypeptide exprimé. Le procédé de transfection est bien connu en soi et désigne l'introduction d'un acide nucléique d'intérêt dans une cellule aux fins de l'expression dudit acide nucléique.

Par "cellules cibles" selon l'invention, on entend les cellules procaryotes, les cellules de levure et les cellules eucaryotes, notamment les cellules animales, et en particulier les cellules de mammifère, notamment les cellules humaines et/ou cancéreuses. *In vivo,* les complexes selon l'invention peuvent être administrés au niveau de l'espace interstitiel ou luminal de tissus tels que le poumon, la trachée, la peau, le muscle, le cerveau, le foie, le coeur, la rate, la moelle osseuse, le thymus, la vessie, la lymphe, le sang, le pancreas, l'estomac, le rein, les ovaires, les testicules, le rectum, le système nerveux périphérique ou central, les yeux, les organes lymphoïdes, les cartilages, l'endothélium.

L'expression d'un gène après transfection à l'intérieur des cellules peut être analysée par des techniques classiques celles que par exemple la détection des ARN messagers par Northern blot ou digestion à la nucléase Si et/ou la détection de protéines par Western blot immunoprécipitation ou test fonctionnel. Ce dernier test est particulièrement adapté lorsque le gène code pour un marqueur protéique tel que par exemple la luciférase ou la β-galactosidase.

L'invention porte aussi sur l'utilisation de complexes tels que décrits précédemment pour la préparation d'un médicament pour le traitement du corps humain ou animal, notamment par thérapie génique. Selon une première possibilité, le médicament peut être administré directement *in vivo* ou par une approche *ex vivo* qui consiste à prélever des cellules du patient, à les transfecter *in vitro* selon l'invention et à les réadministrer audit patient.

L'invention concerne une composition pharmaceutique caractérisée en ce qu'elle comprend au moins un complexe selon la présence invention.

Selon une variante de l'invention, ladite composition pharmaceutique comprend au moins un adjuvant capable d'améliorer le pouvoir de transfection dudit complexe à l'intérieur d'une cellule cible in vitro, ex vivo ou in vivo. Plus particulièrement ledit adjuvant est choisi parmi le groupe consistant en un agent lysosomotropique tel que par exemple la chloroquine, un composé polaire protique choisi notamment parmi le propylène glycol, le polyéthylène glycol, le glycérol, l'éthanol, la 1-methyl L -2-pyrrolidone ou leurs dérivés, et un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés.

Selon le mode d'administration choisi il est également possible d'ajouter à la composition de l'invention un support acceptable d'un point de vue pharmaceutique permettant l'administration à l'homme ou à l'animal. L'utilisation de tels supports est décrite dans la littérature.

Un complexe ou une composition pharmaceutique selon l'invention peuvent être administrés *in vivo* notamment sous forme injectable, notamment par voie intramusculaire. On, peut également envisager une injection par voie intratrachéale, intranasale, épidermique, intraveineuse, intraartérielle, intratumorale, intrapleurale, intracérébrale par seringue ou tout autre moyen équivalent. Selon un autre mode de réalisation, on peut utiliser des systèmes adaptés au traitement des voies aériennes ou des muqueuses tels que l'inhalation, l'instillation, ou l'aérosolisation, par voie topique, par administration orale ou tout autre moyen parfaitement connu de l'homme de l'art et applicable à la présente invention. L'administration peut avoir lieu en dose unique ou répétée, une ou plusieurs fois après un certain délai d'intervalle. La voie d'administration et le dosage les mieux appropriés varient en fonction de différents paramètres tels que par exemple l'individu ou la maladie à traiter, ou encore de l'acide nucléique à transférer ou de l'organe/tissus cible.

L'invention concerne également un polymère cationique de formule I tel que défini précédemment dans lequel le groupe R est choisi parmi le polymethyloxazoline, le polyethyloxazoline, l'acide polyglycolique et les dérivés de cellulose tels que l'hydroxy-methylcellulose ou l'hydroxyethylcellulose

Pris isolément, le polymère cationique selon l'invention contient des monomères portant des fonctions NH₂ libres susceptibles dans des conditions de pH appropriées de devenir NH₃. Dans ces conditions, ledit polymère cationique est capable de former un complexe avec au moins une substance, notamment une substance thérapeutiquement active, comportant des charges négatives.

Enfin, l'invention concerne une cellule transfectée par un complexe ou une composition pharmaceutique tels que définis précédemment, particulièrement une cellule procaryote, une cellule de levure ou eucaryote, notamment une cellule animale, en particulier une cellule de mammifère, et plus particulièrement une cellule cancéreuse.

La présente invention est illustrée par les exemples 1 à S suivants, en référence aux figures 1 à 9.

### LÉGENDES DES FIGURES :

Figure 1 : Elle représente un schéma de synthèse des polyallylamines (PAM.) glycolilées à 50% (PAM/0.5G).

Figure 2 : cartographie du plasmide pTG11033.

Figure 3 : Influence de la charge du complexe ADN/polymère cationique substitué sur l'efficacité de la transfection. L'efficacité de la transfection est traduite par la quantité de luciférase produite (exprimée en RLU/mg de protéine). Les colonnes grisées "clair" représentent les résultats observés en l'absence d'adjuvant de transfection (chloroquine (Cq)), les colonnes grisées "foncé" représentent les résultats observés en présence de 100µM de chloroquine. PAM/0.5G: polymère polyallylamine glycolilée à 50% ; PAM/0.7G: polymère polyallylamine glycolilée à 70%.

Figure 4 : Influence de la quantité d'ADN sur la transfection. Les colonnes grisées "clair" représentent les résultats observés sans chloroquine (Cq), les colonnes grisées "foncé" représentent les résultats observés en présence de 100µM de chloroquine.

Figure 5 : Transfert de gène dans les cellules MRC5. Les colonnes claires représentent les résultats observés avec le PAM/0.5G, les colonnes foncées représentent les résultats observés avec le PAM/0.7G. La présence (Cq) ou l'absence de chloroquine est indiquée en abscisse.

Figure 6 : Structure du PLL/0.7G (polylysine glycolilée à 70%).

Figure 7 : Comparaison des efficacités de transfection de la PAM/0,7G et la PLL/0,7G. PLL : polymère polylysine non substitué ; PLL/0.7G : polymère polylysine glycollilée à 70%.

Figure 8 : Cytotoxicité des polymères cationiques substitués PAM/0.5G et PAM/0.7G.

Figure 9 : Cytotoxicité des complexes ADN plasmidique/polymères cationiques substitués (PAM/0.5G ou PAM/0.7G).

Figure 10 : Influence du taux de substitution du polymère cationique sur l'efficacité de la transfection. Les colonnes noires indiquent que l'expériementation a été conduite en présence de chloroquine, les colonnes blanches en absence de chloroquine.

Figure 11 : Influence de la taille des polymères sur l'efficacité de la transfection, en présence ou en absence de chloroquine. Cq : chloroquine. Les courbes identifiées par ■ figurent les résultats observés avec PAM 0.7G, 60kDa et les courbes identifiées par ▲ figurent les résultats observés avec PAM 0.74G, 10kDa.

Figure 12: Transfert de gène dans les cellules Hela. Les complexes utilisés contiennent du PAM 0,7G à différents équivalents de charge (1,5, 2,5 ou 5 Eq) indiqués en abscisse. Les colonnes claires représentent les résultats observés en absence de chloroquine, les colonnes foncées représentent les résultats observés en présence de chloroquine.

Figure 13 : Effet de l'addition de JTS-1 aux complexes PAM 0.7G sur la transfection de cellules A549. Les quantités de peptide JTS-1 ajoutées sont indiquées en abscisse. Les colonnes blanches figurent les résultats observés en absence de chloroquine et les colonnes noires en présence de chloroquine. L'activité luciférase est exprimée en RLU mesurée sur 15 secondes.

Figure 14: Structure de la polyallylamine éthoxylée

Figure 15: Transfection des cellules A549 avec les PAMs ethoxylées PAM/0.6E ou PAM/0.8E. Les colonnes blanches figurent les résultats observés en absence de chloroquine et les colonnes noires en présence de chloroquine. Les équivalents de charges analysés sont indiqués en abscisse.

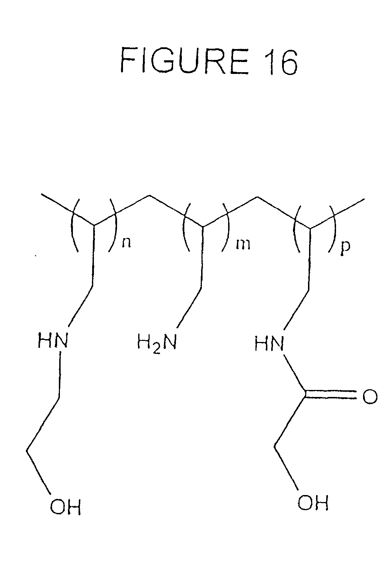

Figure 16: Structure de la polyallylamine glycolylé/éthoxylé

Figure 17:Transfection des cellules A549 avec les PAMs glycolylé 76%/éthoxylé 5 ou 20%. Les colonnes blanches figurent les résultats observés en absence de chloroquine et les colonnes noires en présence de chloroquine. Les équivalents de charges analysés sont indiqués en abscisse.

Figure 18: Injection intraveineuse de PAM/0.7G ou PAM/0.GE dans des souris à un taux de charge fixe de 5.

### EXEMPLES

### A - Matériel et méthodes.

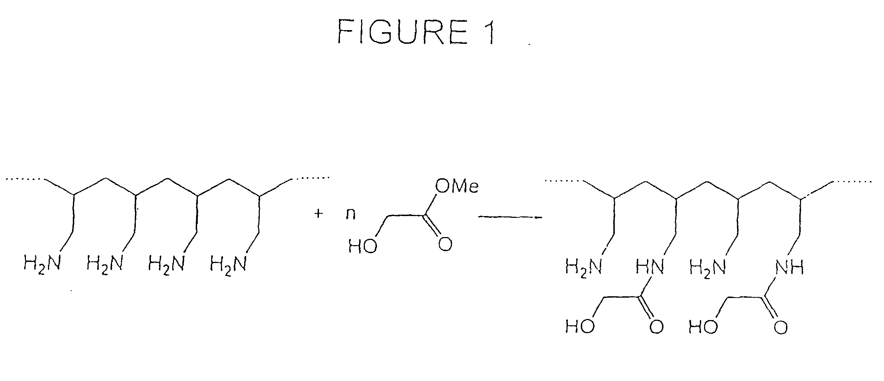

### 1. Synthèse chimique des polyallylamines glycolilées (figure 1)

280 mg de polyallylamine, HCl (PAM, p= 592; SS kDa; soit 3 mmoles de fonction ammonium) [Aldrich] sont dissouts à 67°C pendant environ 10 min dans 1 ml d eau bidistillée. La solution est ensuite placée à température ambiante. 230 µl de glycolate de méthyle (Fluka) (3 mmoles) et 500 µl triéthylamine (TEA) (Fluka) sont ajoutés séquentiellement et agités à température ambiance pendant 72 h. Après évaporation de l'eau sous pression réduite, la masse solide est reprise dans un mélange 50/50 eau bidistillée/ammoniaque 36%, puis soumise à une nouvelle évaporation. Ce traitement est réalisé deux fois afin d'éliminer entièrement la TEA. Le solide enfin obtenu est repris dans (5 ml) de l'eau bidistillée, acidifiée à pH 1 (HCl), puis précipité par addition de 500 ml d'acétone. Le précipité est récupéré par filtration et séché sous vide pendant 12 h. Le polymère est dissout dans (5 ml) d'eau bidistillée puis lyophilisé. Le produit ainsi obtenu est substitué à 50% (PAM/0.5G).

La synthèse de PAM substitué à 70% (PAM/0.7G) ou de polylysine glycolilée à 70% (PLL/0.7G) est réalisée selon une variante de ce protocole pour laquelle on ajoute 6 mmoles de glycolate de méthyle.

Le taux de substitution des différents produits est déterminé par RMN du proton.

### 2 . Les cellules

Les cellules A549 (carcinome pulmonaire) et MRCS (fibroblastes pulmonaires) sont respectivement maintenues en culture dans un milieu DMEM (milieu modifié Dulbecco) ou MEM (milieu essentiel minimum) complémenté avec 10 % de sérum de veau foetal, 286 mg/ml de glutamine et 2 g/l de glucose, dans un incubateur à 37°C, à une atmosphère saturée en humidité contenant 5 % de CO₂. A confluence, les cellules sont détachées du support par traitement à la trypsine-EDTA et réensemencées dans une fiole de culture contenant 15 ml de milieu frais. Environ 18 heures avant l'étape de transfection, les cultures cellulaires sont réparties dans des boites multi-puits, en milieu à 10 % de sérum de veau foetal de façon à obtenir 70-80% de confluence lors de la transfection.

### 3. Le plasmide

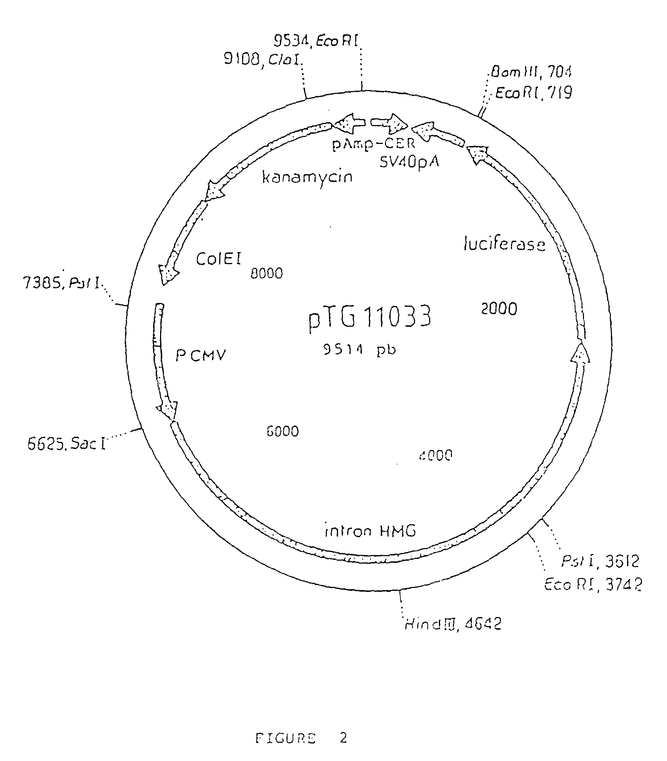

Le polynucléotide choisi, le plasmide pTG11033 (Figure 2), renferme le gène codant pour la luciférase placé sous le contrôle du promoteur CMV, l'intron 1 du gène HMG et le signal de terminaison polyA de SV40.

### 4. Préparation des diverses solutions de polymères PAM/0,5G et PAM/0,7G.

Les concentrations des solutions préparées sont exprimées en millimole de monomère NH₂ non substitué pour un polymère donné. Des solutions 10mM NH₂ sont préparées et stockées à 4°C.
- PAM/0.5G 10 mM (NH₂) : Poids Moléculaire 50860,4
   Une mole de PAM/0.5G correspond à 295,7 moles de fonctions amines NH2 non substituées. 45,5 mg de PAM/0.5G (0,9 µmole et donc 264 µmole de NH₂) sont mis en solutions dans 26,4 mL d'eau bidistillée pour obtenir une solution 10 mM (NH₂).
- PAM/0.7G 10 mM (NH₂): Poids Moléculaire 57720,6
   Une mole de PAM/0.7G correspond à 177,4 moles de fonctions amines NH2 non substituées. Afin d'obtenir une solution 10 mM (NH₂) 58 mg de PAM/0.5G (0,9 µmole et donc 160 µmole de NH₂) sont mis en solutions dans 16 mL d'eau bidistillée.

### 5 . Préparation des complexes ADN/ polymères (PAM/0.5G ou PAM/0,7G).

Dans deux tubes Eppendorf de 1,5 ml, les quantités nécessaires (voir tableau ci-dessous) de polymère et de plasmide sont respectivement diluées dans 150 µl de NaCl ou 150 µl de HEPES 20 mM pH 7,5. La solution est mélangée au vertex puis centrifugée. Après 10 min, les deux solutions sont mélangées et le mélange est placé à température ambiance. Après 10 min d'incubation, les complexes formés peuvent être utilisés pour la transfection de cellules.

| | | |
|---|---|---|
| nombre d'équivalents (eq.) | 2,5 | 5 |
| Volume de solution | 1,5 µl/2 µg* | 3 µl/2 µg* |
| de polymère 10 mM (NH₂) | 3 µl/4 µg* | 6 µl/4 µg* |

| | | |
|---|---|---|
| * : Quantité de plasmide. | | |

La proportion d'amines protonées du polymère à pH donné, n'étant pas connue de manière exacte, les complexes analysés sont exprimés en équivalents (eq) d'azote par rapport au phosphate de l'ADN (N/P). Ainsi un équivalent représentera la quantité de polymère nécessaire pour avoir un azote par groupement phosphate de l'ADN (Felgner et al.,1997, Human Gene Therapy, 8, 511-512).

### 6. Transfection

100 µl du complexe obtenu au point 5 sont versés sur les cellules distribuées en multi-puits comme décrit précédemment, préalablement rincées avec du milieu frais sans sérum. Après 2 à 3 h, le milieu est substitué par du milieu complété avec 10% de Sérum de Veau Foetal. Le taux d'expression du gène luciférase est mesuré après 48 h. Pour cela, les cellules transfectées sont lysées dans un tampon de lyse (Promega). Les débris cellulaires sont ensuite éliminés par centifugation et l'activité luciférase (en RLU/min, Unité de lumière relative par minute) est mesurée sur 20 µl de surnageant conformément aux instructions du fournisseur (Promega) en ajoutant 100 µl de réactif et en mesurant l'activité par luminescence. La quantité de protéine totale est, par ailleurs, déterminée par la méthode colorimétrique d'acide bicinchoninique BCA (Smith et al., 1985, Anal. Biochem., 150, 76-85 Pierce) à partir d'un aliquote de surnageant. Ceci permet d'exprimer l'activité luciférase en RLU par milligramme de protéine extraite des cellules.

### 7. Transfection en présence de chloroquine 100 µM:

Selon une variante de la technique de transfection proposée ci-dessus, Le volume nécessaire d'une solution 10 mM de chloroquine est ajouté au milieu de culture juste avant l'addition du complexe ADN/polymère de manière à obtenir une concentration finale par puits de 100 µM.

### B - RÉSULTATS

### Exemple 1: Transfert de gène dans les cellules A549 et influence de la charge du complexe ADN/polymère cationique.

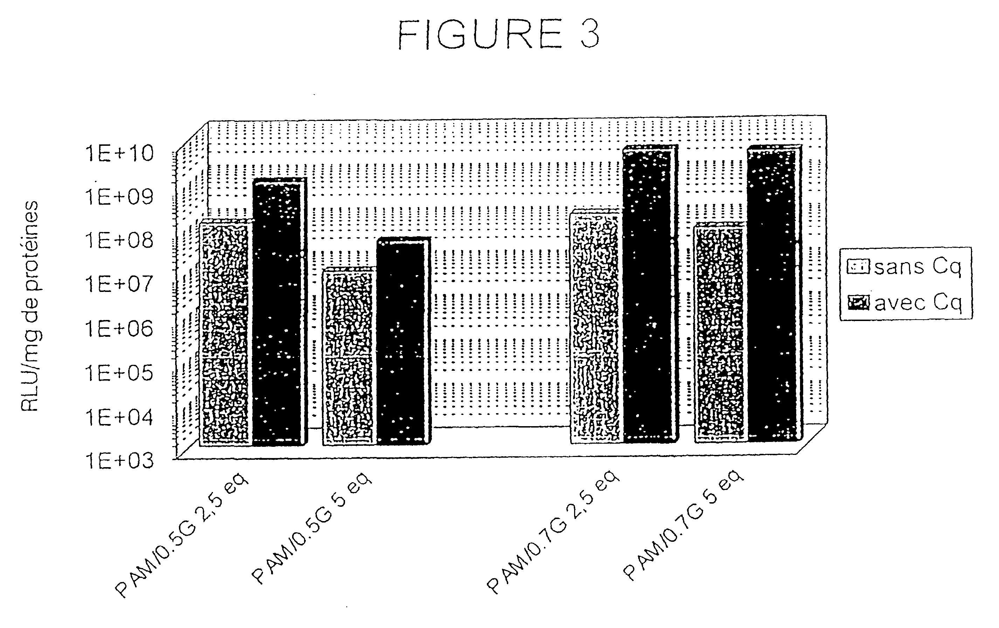

Dans un premier temps, différents complexes sont formés selon le protocole exposé plus haut en ajoutant deux quantités différentes de PAM/0,5G 10mM ou de PAM/0,7G 10 mM à une quantité fixe de plasmide (2µg), afin d'obtenir différents ratios N/P (équivalents ; 2.5 ou 5 eq). Ceci permet d'étudier si la charge totale du complexe formé par interactions éléctrostatiques entre les aminés du polymère cationique analysé et les phosphates de l'ADN plasmidique joue un rôle sur l'efficacité de la transfection. Les résultats (figure 3) montrent que les PAMs glycolilées à 50% ou à 70% sont capables de transférer efficacement l'ADN dans les cellules, même à faible équivalent de charge. On peut par ailleurs noter que la présence de chloroquine (100 µM) permet d'améliorer l'efficacité de transfection.

### Exemple 2: Effet de la quantité d'ADN sur l'efficacité de la transfection.

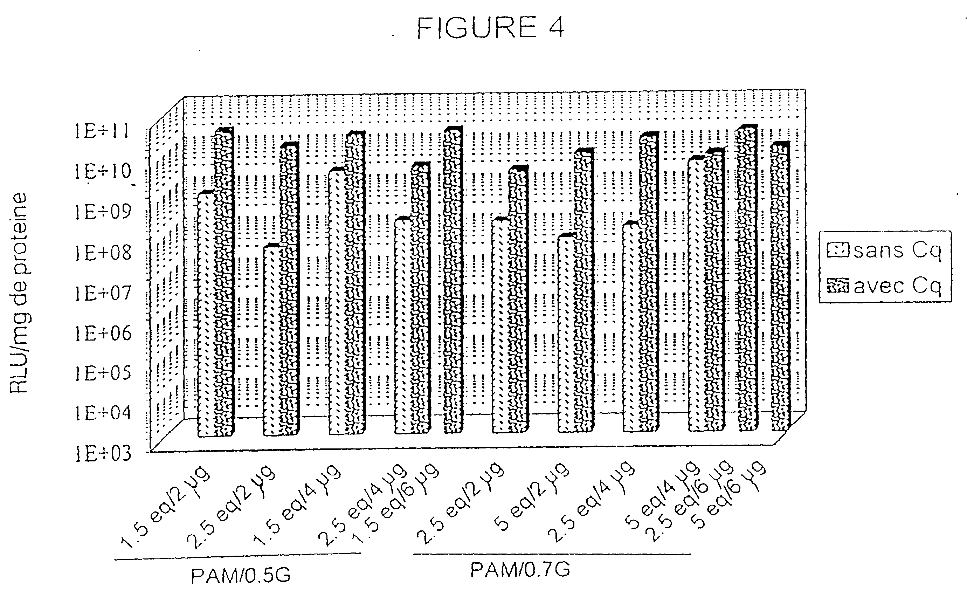

Les complexes pour cette série d'expériences sont formés en mélangeant des quantités croissantes d'ADN plasmidique (2, 4 ou 6 µg) et des quantités variables de PAM/0,5G ou de PAM/0,7G afin d'obtenir des ratios N/P de 1.5 eq, 2.5 eq ou 5 eq. Les résultats obtenus (figure 4) après transfection des cellules A549 avec ces différents complexes montrent que les polymères PAM/0,5G et PAM/0,7G permettent une transfection efficace des cellules, avec ou sans chloroquine, quelle que soit la quantité d'ADN. Il est en outre intéressant de noter que la chloroquine n'est pas indispensable pour obtenir un taux d'efficacité performant lorsque l'on utilise pour la transfection un complexe ADN/PAN/0,7G préparé à partir de 4 µg d'ADN et à 5 équivalents de charge.

### Exemple 3: Transfert de gène dans les cellules MRCS

Il est connu que l'efficacité du transfert de gène par utilisation de vecteurs non-viraux peut varier notamment en fonction du type cellulaire considéré. Nous avons donc testé notre système de transfection sur un autre type cellulaire tumoral, les MRCS, considérées comme des cellules difficilement transfectables (Boussif et al., 1996, Gene Therapy, 3, 1010 - 1017). Les complexes ont été préparés comme décrit précédemment en mélangeant des quantités variables d'ADN plasmidique (2 ou 4 µg) et des quantités variables de polymères cationiques PAM/0,5G ou PAM/0,7G de façon à obtenir des valeurs d'équivalents de charge de 2.5 ou de 5 eq. Par ailleurs, les transfections ont été réalisées en présence ou en l'absence de chloroquine (100µM).

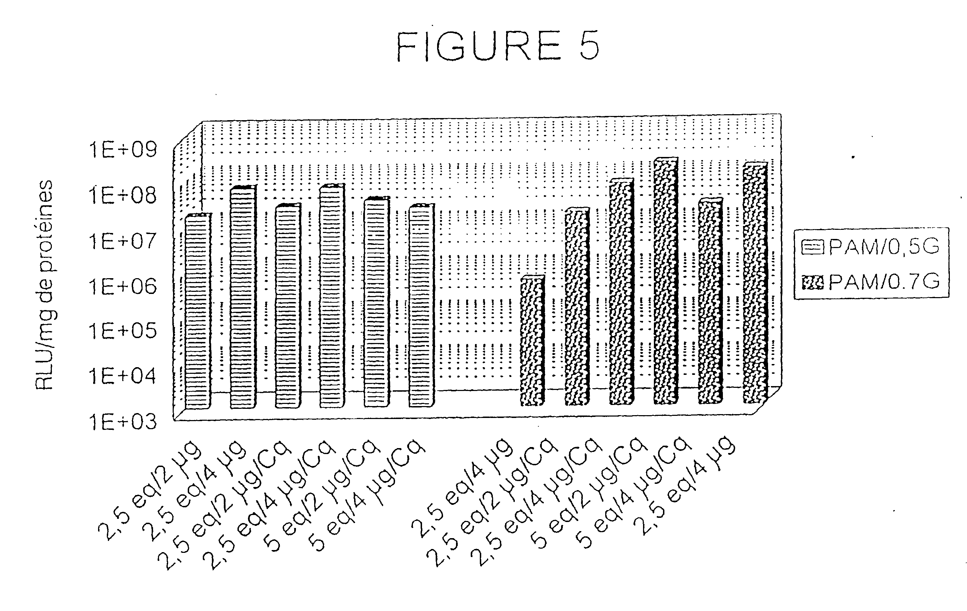

Les résultats présentés à la figure 5 montrent que les cellules MRC5 sont transfectées efficacement dans les conditions expérimentales testées.

### Exemple 4: Comparaison des efficacités de transfection des PAM/0,7G et PLL/0.7G

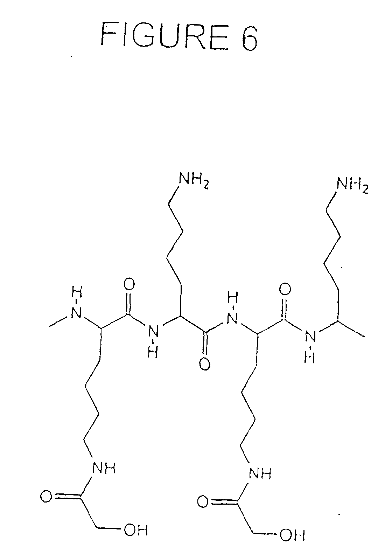

Afin de montrer l'importance de la nature chimique du polymère, les efficacités de transfection du PAM/0,7G à celles observées avec un analogue obtenu à partir de la polylysine (PLL) dont 70% des fonctions NH₂ libres ont été glycolilées comme décrit en A.1. (Figure 6).

La transfection des cellules A549 a été réalisée comme décrit précédemment en utilisant des complexes ADN/polymère cationique pour lesquels ledit polymère est du PAM/0,7G ou du PLL/0,7G ou de la polylysine non susbstituée. En outre, ces complexes ont été préparés avec différences quantités d'ADN (2 ou 4µg), et en faisant varier le rapport N/P (2.5 ou 5 eq).

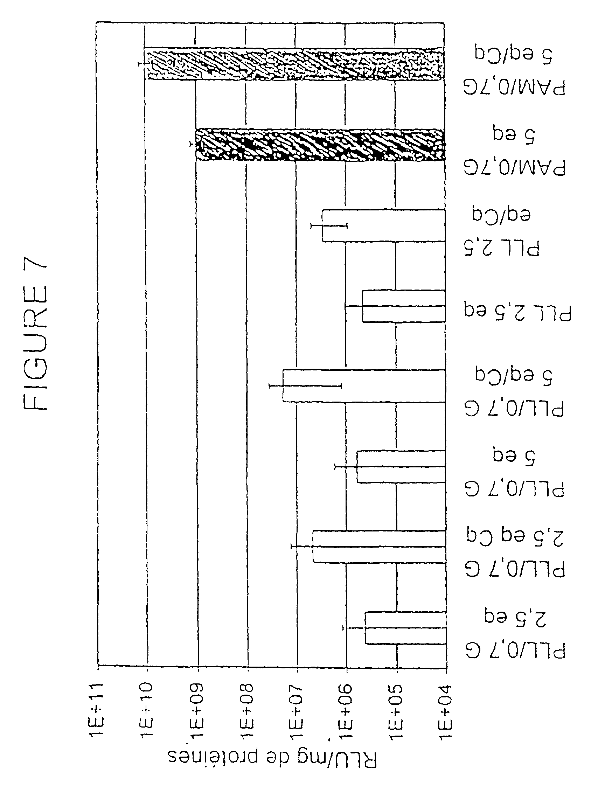

Les résultats obtenus (figure 7) montrent que la substitution à 70% du polymère PLL ne permet pas d'améliorer les faibles taux d'expression observés avec la polylysine non-modifiée (PLL) qui sont de 50 à 100 fois plus faibles que ceux observés dans des conditions identiques avec la PAM/0,7G.

### Exemple 5 : Cytotoxicité des polymères et des complexes ADN/Polymère

La cytoxicité des polymères PAM substitué et non substitué a été analysée en réalisant un test colorimétrique au MTT (bromure du 3-(4,5-dimethylthiazol-2-yl)-2,5-diphenyltetrazolium)(Mossman T., 1983, Journal of Immunological Methods, 65, 55-63).

Dans un premier temps, les cellules A549 en culture sont placées en présence de quantités croissantes de polymères non complexé. Ces quantités correspondent aux quantités nécessaires pour former des complexes avec 4 µg d'ADN et pour obtenir des rapports N/P allant de 2,5 à 30 eq.

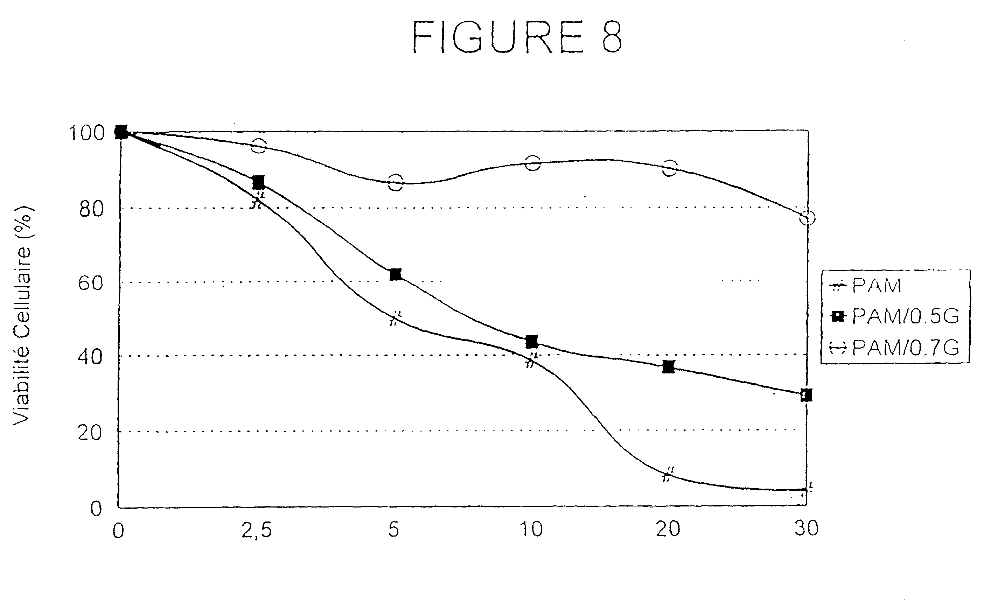

Les résultats (figure 8) montrent que les PAM/0,5G et PAM/0,7G sont beaucoup moins toxiques à l'égard des cellules que le polymère PAM non substitués (Remarque: 100% de viabilité correspond aux cellules n'ayant subi aucun traitement).

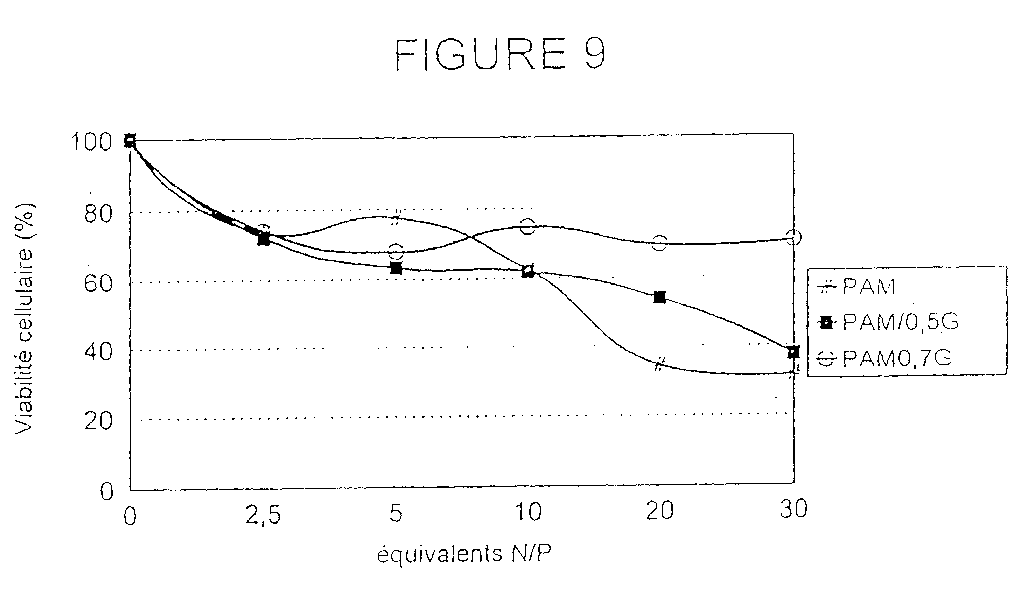

Dans un second temps, une série d'expériences similaires a été réalisée avec les mêmes polymères sous une forme de complexe polymères cationique/ADN (4µg de plasmide). Les taux de viabilité cellulaires présentées sur la figure 9 confirment que les complexes polymère cationique/ADN selon l'invention sont très faiblement toxiques à l'égard des cellules.

La viabilité cellulaire observée avec la PAM/0,7G à 30 équivalents est supérieure à 70%. Ceci est très prometteur pour les essais *in vivo* pour lesquelles les quantités d'ADN et par conséquent de polymère, à utiliser peuvent être importantes.

### Exemple 6: Transfert de gène dans les cellules A549 et influence du taux de substitution du polymère cationique.

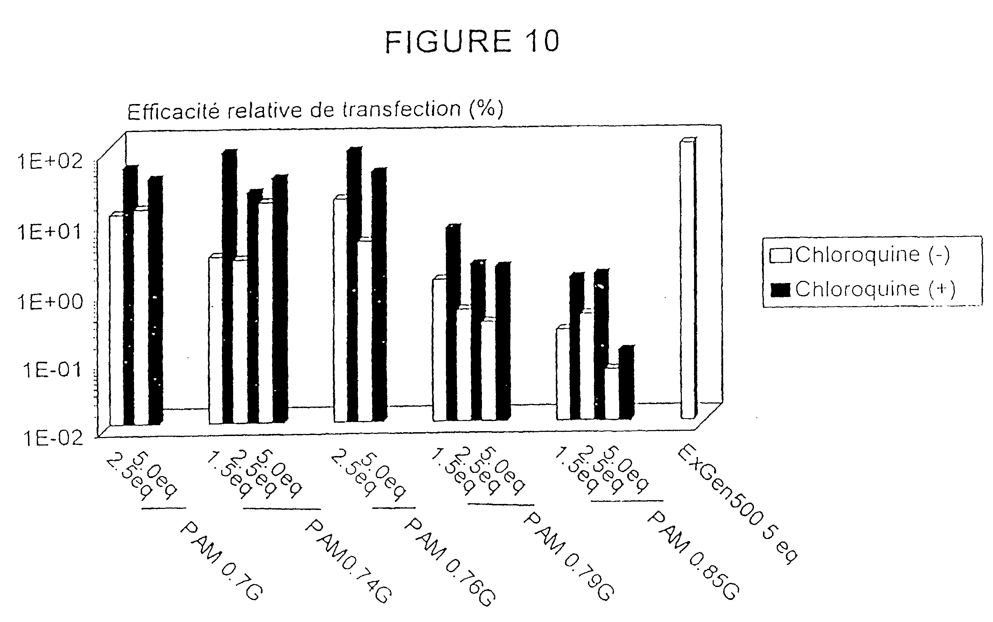

Différents polymères cationiques selon l'invention sont préparés donc les taux de substitution varient de 70 à 85 %. Conformément aux protocoles exposés plus haut, plusieurs complexes sont formés à partir d'une quantité fixe de plasmide pTG11033 (2µg) et de ces différents polymères cationiques (0.7G, 0.74G, 0.76G, 0.79G, 0.85G) de façon à obtenir différents ratios N/P (équivalents ; 1.5, 2.5 ou 5 eq). Ceci permet d'étudier si le taux de substitution du polymère joue un rôle sur l'efficacité de transfection. Pour chaque série, cinq expériences indépendantes ont été réalisées. Par ailleurs, le polymère ExGen500 (Euromedex, Souffelweirsheim, France) complexé à 2µg de plasmide est utilisé à titre,de standard. Les résultats (figure 10) montrent que les PAMs glycolilées de 70% à environ 76% sont capables de transférer efficacement l'ADN dans les cellules, même à faible équivalent de charge, et qu'au delà (79 et 85%), une décroissance de l'efficacité est observée, avec ou sans chloroquine (100 µM).

### Exemple 7; Effet de la taille des polymères.

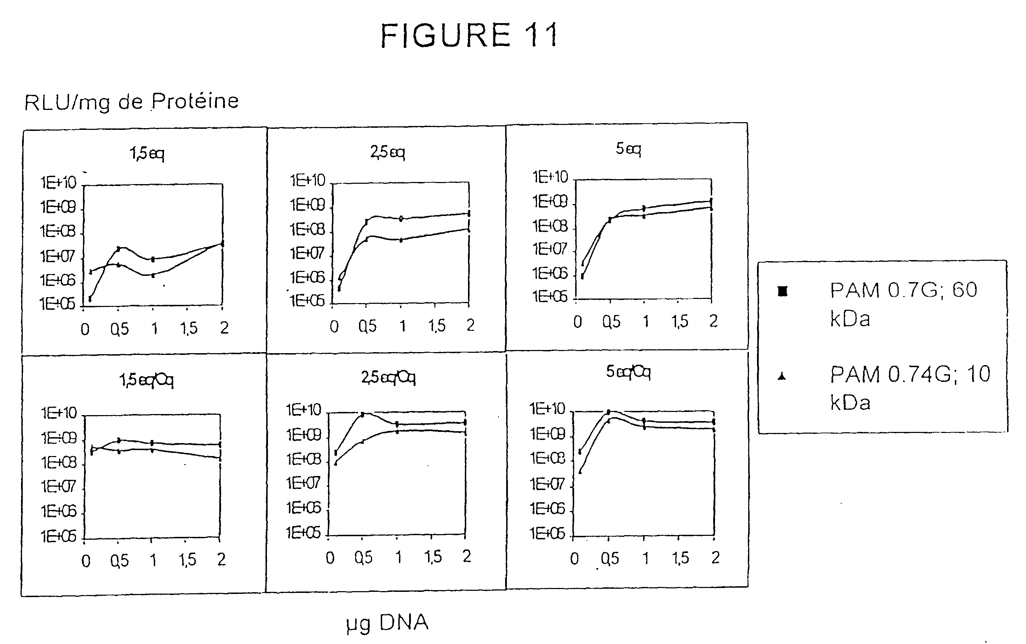

Des polymères cationiques selon l'invention, avec deux degrés différents de polymérisation (60 kDa et 10 kDa Aldrich) présentant des taux de substitution comparables (74% et 70%) sont préparés. Des complexes sont formés selon les protocoles décrits précédemment pour lesquels la quantité de plasmide complexé varie de 0.1 à 2µg, les ratios N/P varient de 1.5, 2.5 à 5 eq. Les résultats obtenus (figure 11) après transfection des cellules A549 avec ces différents complexes, en présence ou en absence de chloroquine, montrent que la taille du polymère utilisé n'influence pas sa capacité à transfecter les cellules.

### Exemple 8 : Transfert de gène dans les cellules HeLa

Nous avons également testé notre système de transfection sur un autre type cellulaire, les cellules HeLa, lignée dérivée d'un carcinome du cervix humain. Les complexes ont été préparés comme décrit précédemment en mélangeant 2 µg d'ADN plasmidique et des quantités variables de polymères cationiques PAM/0,7G de façon à obtenir des valeurs d'équivalents de charge de 1.5, 2.5 ou de 5 eq. Par ailleurs, les transfections ont été réalisées en présence ou en l'absence de chloroquine (100µM).

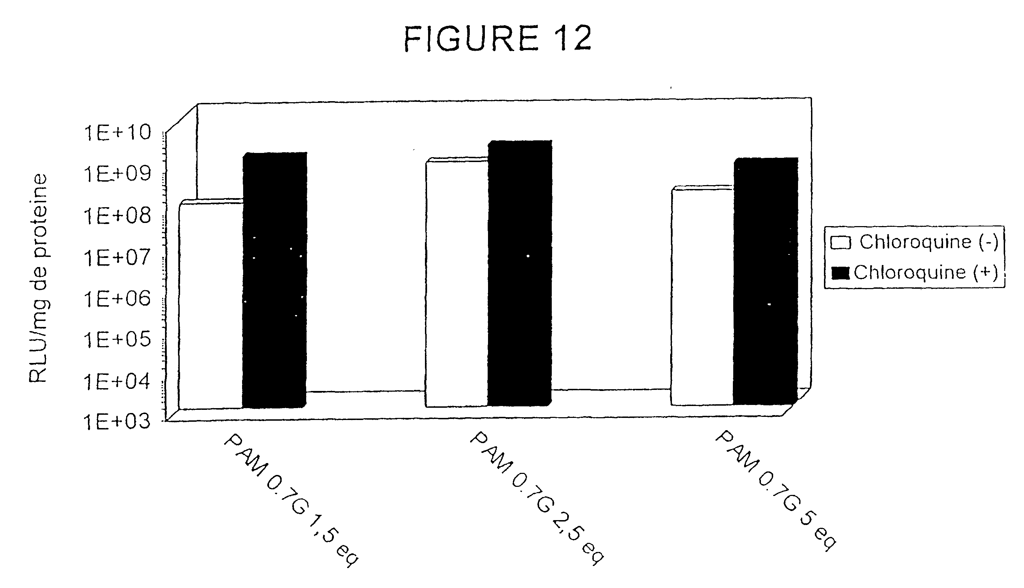

Les résultats présentés à la figure 12 montrent que les cellules HeLa sont transfectées efficacement dans les conditions expérimentales testées.

### Exemple 9: Influence d'un peptide disrupteur d'endosome sur l'efficacité de la transfection.

Dans cet exemple, nous avons réalisé des expériences de transfection de complexes pTG11033/PAM0.7G, 2.5 eq ou pTG11033/PAM 0.7G, 5 eq en association avec un peptide disrupteur d'endosome (peptide JTS1 décrit par Gottschalk et al., 1996, Gene Therapy, 3, 448-457).

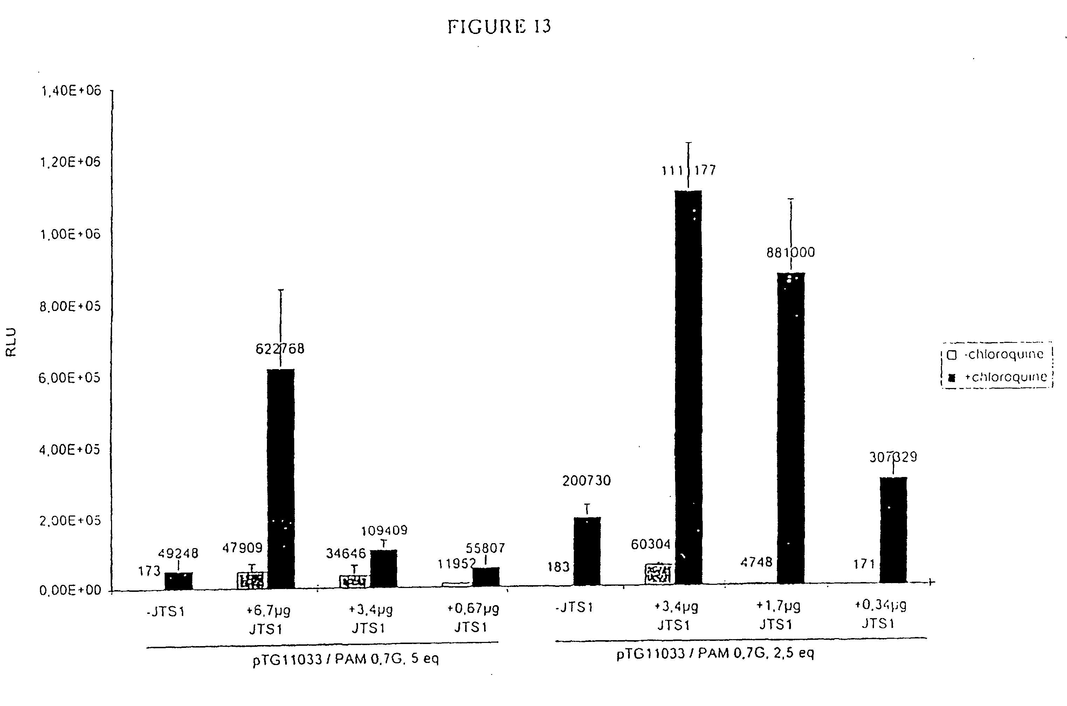

Environ 24 heures avant la transfection, des plaques "24 puits" sont ensemencées avec 10⁵ cellules A549 par puits de 1 ml. Le milieu de culture est le DMEM + glutamine + gentamycine + 10% de sérum de veau foetal. Le lendemain, différences quantités de JTS1 (voir figure 13) sont ajoutées à 20µl de complexes pTG11033/PAM 0.7G, 5 eq ou pTG11033/PAM 0.7G, 2.5 eq (ces complexes sont préparés comme décrits précédemment et renferment 2µg de pTG11033). Après 30 minutes d'incubation à température ambiante, les mélanges sont dilués dans du milieu DMEM sans sérum de manière à obtenir une concentration finale de plasmide de 1µg/ml. 200µl de cette préparation (soit l'équivalent de 200 ng de plasmide) sont déposés sur les cellules (pour chacun des complexes, l'expérience est reproduite sur 3 puits différents) en absence ou en présence de chloroquine (100µM final) après aspiration du milieu de culture. 800µl de milieu contenant 10% de sérum sont ajoutés 3 à 4 heures après la transfection.

L'expression du gène codant pour la luciférase est mesurée 24 heures après la transfection. Les cellules transfectées sont lysées dans 100µl de tampon de lyse (Proméga) et congelées à -80°C. Aprés dégel, 20µl d'extraits sont prélevés pour le dosage de la luciférase. Un luminomètre Microlumat (Berthold Microlumat LB96P) est utilisé pour quantifier la production de luciférase. 100µl de réactif (Luciferase assay system, Proméga) sont ajoutés sur les extraits et la luminescence exprimée en RLU est mesurée pendant 15 sec.

Les résultats (figure 13) montrent que l'addition de JTS1 permet d'augmenter l'efficacité de transfection d'environ 250 à 300 fois en absence de chloroquine. En présence de chloroquine, l'efficacité de transfection des complexes PAM 0.7G est fortement augmentée. L'addition de JTS1 permet d'augmenter l'efficacité de transfection de 5 (PAM 0.7G, 2.5 eq) à 13 fois (PAM 0.7G, 5 eq).

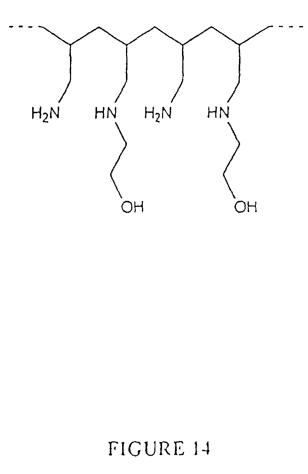

### Exemple 10 : Synthèse chimique des polyallylamine éthoxylées PAM/0,6E et PAM/0,8E (figure 14).

Conformément à la présente invention, nous avons préparé des polyallylamines substituées par un autre groupe hydrophile. Pour ce faire, 0.2854 g (2.762 mmoles de fonction amine) de PAM-HCl (Aldrich-28,322-3) en solution dans 0.5 ml d'eau milliporée sont ajoutés à 198 ml d'iodoéthanol (0.92 équivalents - 2.541 mmoles). Sont ensuite ajoutés a) 2 ml de DMSO afin de satisfaire aux contraintes de solubilité de l'iodoéthanol et b) 0.74 ml de triéthylamine (5.524 mmoles). Après une agitation à 30°C pendant 72h00, le milieu est évaporé sous vide, et trois co-évaporations avec de l'ammoniaque à 18% permettent d'éliminer la triéthylamine. Le résidu est repris dans l'eau puis est acidifié par ajout d'HCl concentré. Le volume final de la phase aqueuse est 60 ml. Trois extractions avec 20 ml d'éther sont réalisées afin d'éliminer la majorité de l'iodoéthanol résiduel. La phase aqueuse est concentrée au Rotavap™ puis précipitée dans 600 ml d'acétone. Après filtration sur fritté, le solide est repris dans l'eau. Après dialyse contre plusieurs bains de 4 l d'eau milloporée, l'échantillon est lyophilisé. Le degré de fonctionnalisation est déterminé par RMN du proton. Selon les conditions décrites ci-dessus, 60% des fonctions amines primaires du polymère sont ethoxylées. De façon identique, le protocole a été adapté de manière à obtenir des polymères dont 80% des fonctions amines primaires sont éthoxylées.

### Exemple 11 : Transfection des cellules A549 avec les PAMs ethoxylées PAM/0,6E ou PAM/0,8E.

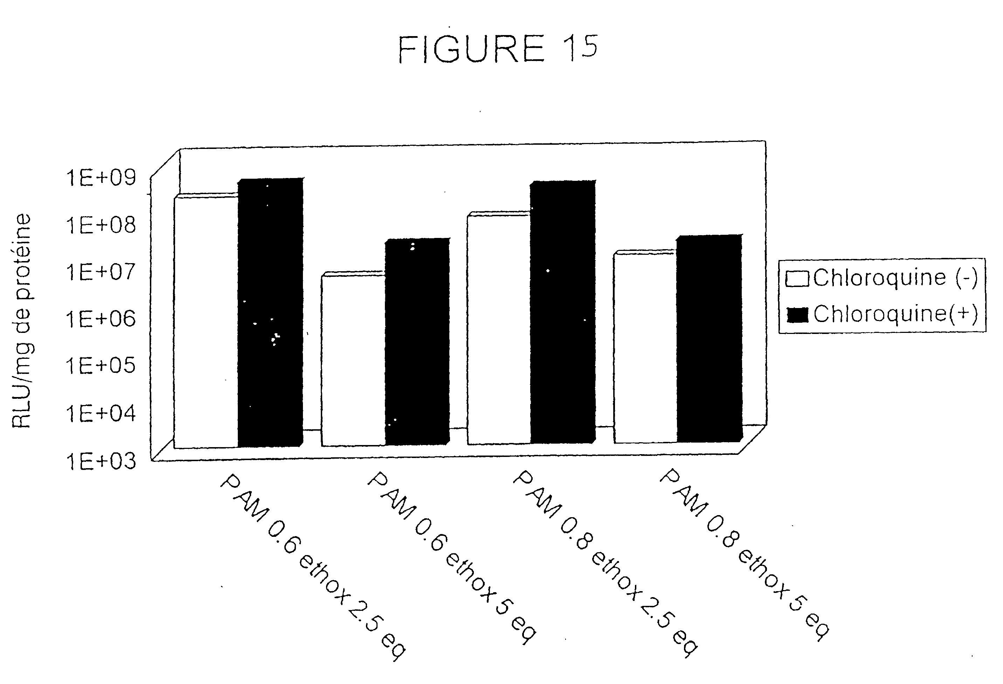

Les complexes ADN/polymères (PAM/0.6E ou PAM/0.8E) sont préparés dans les mêmes conditions que celles précédemment décrites pour la préparation des complexes comprenant des PAM glycolylées, en particulier les équivalents de charges sont définis de la même façon (amine potentiellement protonable/Phosphate de l'ADN). Conformément au protocole décrit pour les PAM glycolylées, les cellules A549 sont transfectées avec 2 µg de plasmide pTG11033 complexé à la quantité désirée de polymère, en présence ou en absence de chloroquine (100 µM finale). L'activité luciférase est mesurée après 48 heures d'incubation. Les résultats (figure 15) confirment que la substitution des polyallylamines par un groupe de nature hydrophile permet d'observer un taux de transfection efficace, en présence et en absence de chloroquine.

### Exemple 12 :Transfection des cellules A549 avec les PAMs à la fois ethoxylées et glycolylées : PAM/0,76G/0,05E ou PAM/0,76G/0,2E.

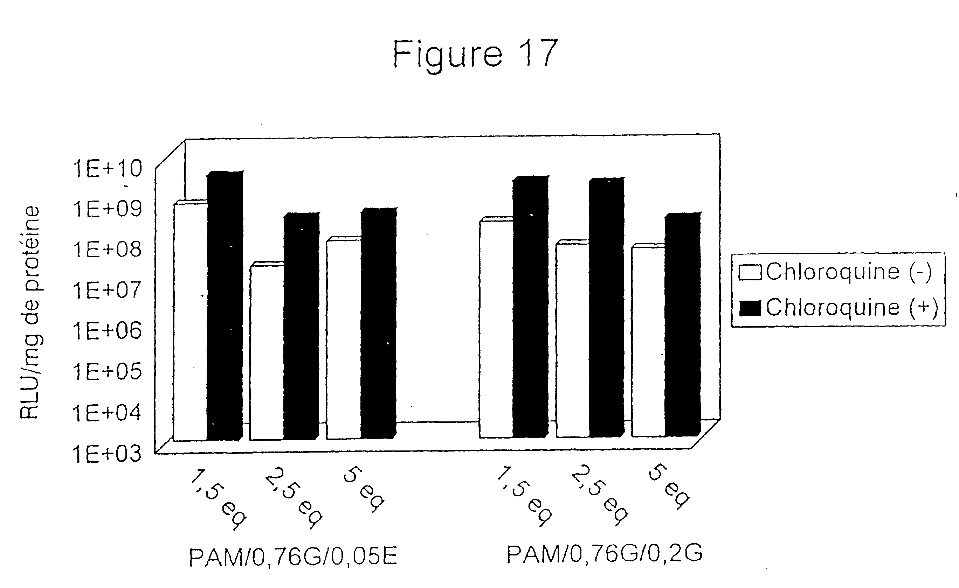

Les complexes ADN/polymères (PAM/0.76G/0.05E ou PAM/0.76G/0.2E) sont préparés dans les mêmes conditions que celles précédemment décrites pour la préparation des complexes comprenant des PAM glycolylées, en particulier les équivalents de charges sont définis de la même façon (amine potentiellement protonable/Phosphate de l'ADN). Conformément au protocole décrit pour les PAM glycolylées, les cellules A549 sont transfectées avec 2 µg de plasmide pTG11033 complexé à la quantité désirée de polymère, en présence ou en absence de chloroquine (100 µM finale). L'activité luciférase est mesurée après 48 heures d'incubation. Les résultats (figure 17) confirment que la substitution des polyallylamines par un groupe de nature hydrophile permet d'observer un taux de transfection efficace, en présence et en absence de chloroquine.

### Exemple 13 :Injection intraveineuse de PAM glycolylée PAM/0.7G ou de PAM éthoxylée PAM/0.6E dans des souris

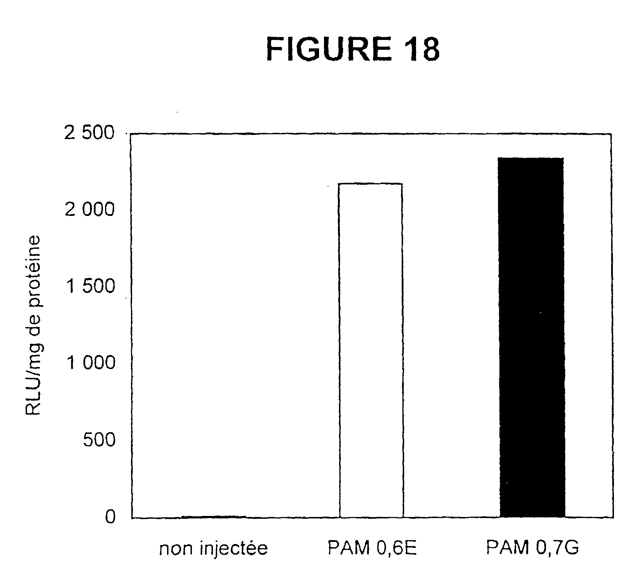

Les résultats sont reportés sur la figure 18. Des complexes ADN/polymère selon l'invention ont été synthétisés dans une solution 5% glucose selon les méthodes décrites précédemment à partir du polymère cationique PAM 0.7G ou le polymère cationique PAM 0.6E, à un ratio de charge fixe de 5, en utilisant un plasmide contenant le gène de la luciférase pTG11033 (demande de brevet français n°97/08267).

Les souris utilisées sont des souris femelles C57BL/6 de 6 semaines . Les injections intraveineuses sont effectuées dans la queue après désinfection de la peau à l'éthanol 70%. Le volume injecté est 250µl et la concentration en ADN est 0.24 mg/ml. Un jour après les injections, les souris sont sacrifiées. Après extraction, les tissus sont congelés dans de l'azote liquide. Afin de mesurer l'activité luciférase, les tissus sont mis dans 200 ou 500 µl de tampon de lyse (Promega) et puis broyés à l'aide d'un homogénéiseur Polytron (Kinenatica). Les tissus sont soumis à une homogénéisation de 2 x 30 s et pendant les premières 5 s la vitesse de rotation du Polytron est augmentée graduellement jusqu'au maximum (position 11 sur l'appareil). L'homogénat ainsi obtenu à partir des poumons est soumis à trois étapes de congélation/décongélation. Les débris cellulaires sont culotés par centifugation et l'activité luciférase (en RLU/min, Unité de lumière relative par minute) est mesurée sur 20 µl de surnageant conformément aux instructions du fournisseur (Promega) en ajoutant 100µl de réactif et en mesurant l'activité par luminescence (luminomètre Berthold 9500). La quantité de protéine totale est, par ailleurs, déterminée par la méthode colorimétrique d'acide bicinchoninique BCA (Smith et al., 1985, Anal. Biochem., 150, 76-85 Pierce) à partir d'un aliquote de surnageant. Ceci permet d'exprimer l'activité luciférase en RLU par milligramme de protéines extraites des tissus. Des souris non injectées ont été utilisées comme témoin négatif.

Les résultats montrent une expression du gène rapporteur luciférase dans les poumons après injection intraveineuse de complexes tels que décrits ci-dessus par rapport aux souris non injectées. Les valeurs indiquées sont des valeurs moyennes obtenues à partir de 2 (non injectées), 3 (PAM 0.7G) et 1 (PAM 0.6 E) souris injectées.

## Revendications

1. Complexe comprenant au moins un polymère cationique de formule : 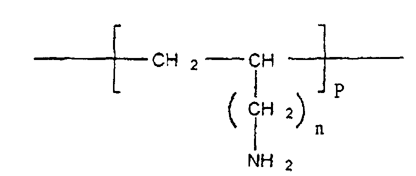 pour lequel n est un nombre entier variant de 0 à 5 et p est un nombre entier variant de 2 à 20000, plus particulièrement p varie de 10 à 18000, et avantageusement de 200 à 1000,
**caractérisé en ce que** :
- au moins 10%, avantageusement de 30 à 80%, préférentiellement 70%, des fonctions NH₂ libres sont substituées par des groupes R hydrophiles identiques ou différents,
- ledit polymère cationique peut en outre comprendre au moins un élément de ciblage associé de manière covalente ou non' aux fonctions NH₂ libres et/ou auxdits groupes R hydrophiles sous réserve que ledit polymère cationique contienne au moins 20%, préférentiellement au moins 30%, de fonctions NH₂ libres, associé à au moins une substance thérapeutiquement active, comportant au moins une charge négative.

2. Complexe selon la revendication 1, **caractérisé en ce que** ledit polymère cationique répond à la formule : 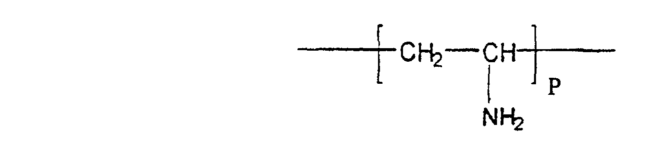 (n=0)

3. Complexe selon la revendication 1, **caractérisé en ce que** ledit polymère cationique répond à la formule : 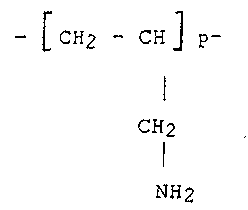 (n=1)

4. Complexe selon l'une quelconque des revendications 1 à 3 **caractérisé en ce que** lesdits groupes hydrophiles R dudit polymère cationique comprennent au moins une fonction hydrophile choisie parmi les fonctions amine, hydroxyle, amide et ester.

5. Complexe selon l'une quelconque de revendications 1 à 4 **caractérisé en ce que** R est choisi parmi les groupes R'-C=0 et -(CH₂)ₙ,-R', où R' désigne un groupement contenant au moins une fonction hydrophile n' est un nombre entier variant de 1 à 5.

6. Complexe selon l'une quelconque des revendications 1 à 4, **caractérisé en ce que** R ou R' consiste en un polymère présentant des propriétés hydrophiles.

7. Complexe selon la revendication 6, **caractérisé en ce que** R ou R' est choisi parmi le polyéthylène glycol et ses dérivés, la polyvinylpyrrolidone, le polymethyloxazoline, le polyethyloxazoline, le polyhydroxypropyl methacrylamide, l'acide polylactique, l'acide polyglycolique et les dérivés de celluloses tels que l'hydroxymethylcellulose ou l'hydroxyethylcellulose.

8. Complexe selon la revendication 7, **caractérisé en ce que** R ou R' est le polyéthylène glycol (PEG) ayant un poids moléculaire variant de 300 à 5000, plus particulièrement de 1000 à 4000 et avantageusement ayant un poids moléculaire de 2000 (PEG 2000).

9. Complexe selon l'une des revendications 3 ou 4, **caractérisé en ce qu'**il s'agit d'une polyallylamine glycolilée.

10. Complexe selon la revendication 9, **caractérisé en ce que** R est 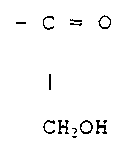 et environ 50 à environ 70 % de fonctions NH₂ libres sont substituées par R.

11. Complexe selon la revendication 3 ou 4, **caractérisé en ce qu'**il s'agit d'une polyallylamine éthoxylée.

12. Complexe selon la revendication 11, **caractérisé en ce que** R est (CH₂)₂₋OH
et environ 50 à environ 80 de fonctions NH₂ libres sont substituées par R.

13. Complexe selon la revendication 10 ou 12, **caractérisé en ce que** p = 592.

14. Complexe selon l'une quelconque des revendications 1 à 13 **caractérisé en ce que** ledit élément de ciblage est présent dans ledit polymère cationique et **en ce qu'**il est choisi parmi le groupe consistant en tout ou partie de sucres, de peptides, d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogèniques, de peptides de localisation nucléaire, ou d'une combinaison de tels composés.

15. Complexe selon l'une des revendications 1 à 14, **caractérisé en ce que** la substance thérapeutiquement active est choisie parmi les acides nucléiques et les protéines.

16. Complexe selon la revendication 15 **caractérisé en ce que** ladite substance thérapeutiquement active est un acide nucléique choisi parmi le groupe consistant en un cADN, un ADN génomique, un ADN plasmidique, un ARN messager, un ARN antisens, un ribozyme, un ARN de transfert, un ARN ribosomique, ou un ADN codant pour de tels ARN.

17. Complexe selon la revendication 16 **caractérisé en ce que** ledit acide nucléique comprend un gène d'intérêt et des éléments d'expression dudit gène d'intérêt.

18. Complexe selon la revendication 17, **caractérisé en ce que** ledit gène d'intérêt code pour tout ou partie d'un ribozyme, d'un acide nucléique antisens, d'un polypeptide, notamment thérapeutiquement actif ou marqueur.

19. Complexe selon l'une des revendications 1 à 18, **caractérisé en ce que** le rapport entre le nombre de charges positives dudit polymère cationique et le nombre de charges négatives de ladite substance thérapeutiquement active varie de 1 à 30, de préférence de 1,5 à 10 et avantageusement de 2,5 à 5.

20. Procédé de préparation d'un complexe selon l'une des revendications 1 à 19, **caractérisé en ce que** l'on met en présence un ou plusieurs polymères cationiques de formule : 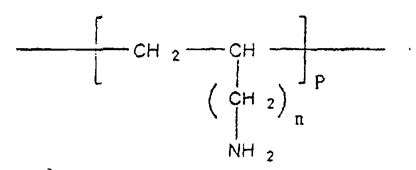 pour lequel n est un nombre entier variant de 0 à 5 et p est un nombre entier variant de 2 à 20000, plus particulièrement p varie de 10 à 18000, et avantageusement de 200 à 1000, **caractérisé en ce que** :
- au moins 10%, avantageusement de 30 à 80%, préférentiellement 70%, des fonctions NH₂ libres sont substituées par des groupes R hydrophiles identiques ou différents,
- ledit polymère cationique peut en outre comprendre au moins un élément de ciblage associé de manière covalente ou non aux fonctions NH₂ libres et/ou auxdits groupes R hydrophiles sous réserve que ledit polymère cationique contienne au moins 20%, préférentiellement au moins 30%, de fonctions NH₂ libres, avec une ou plusieurs substances thérapeutiquement actives comportant au moins une charge négative et **en ce que** l'on récupère ledit complexe éventuellement après une étape de purification.

21. Procédé pour transférer une substance thérapeutiquement active dans une cellule cible in vitro, **caractérisé en ce que** l'on met en contact des cellules cibles avec au moins un complexe selon l'une quelconque des revendications 1 à 19.

22. Procédé selon la revendication 21, **caractérisé en ce que** ladite cellule cible est choisie parmi les cellules procaryotes, les cellules de levure, les cellules eucaryotes, notamment les cellules animales, et plus particulièrement les cellules de mammifères, notamment les cellules humaines et/ou cancéreuses.

23. Utilisation d'un complexe selon l'une des revendications 1 à 19 pour la préparation d'un médicament pour le traitement du corps humain ou animal, notamment par thérapie génique.

24. Utilisation selon la revendication 23, **caractérisée en ce que** le médicament est destiné à être administré sous forme injectable, notamment par voie intramusculaire.

25. Utilisation selon la revendication 23, **caractérisée en ce que** le médicament est destiné à être administré par inhalation, par instillation, par aérosolisation, par voie topique ou par voie orale.

26. Composition pharmaceutique **caractérisée en ce qu'**elle comprend au moins un complexe selon l'une quelconque des revendications 1 à 19.

27. Composition selon la revendication 26 **caractérisée en ce qu'**elle comprend au moins un adjuvant capable d'améliorer le pouvoir de transfection dudit complexe à l'intérieur d'une cellule cible in vitro, ex vivo ou in vivo.

28. Composition selon la revendication 27, **caractérisée en ce que** ledit adjuvant est choisi parmi le groupe consistant en un agent lysosomotropique tel que la chloroquine, un composé polaire protique notamment choisi parmi le propylène glycol, le polyéthylène glycol, le glycérol, l'éthanol, la 1-methyl L -2-pyrrolidone ou leurs dérivés, et un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés.

29. Composition selon l'une des revendications 26 à 28 **caractérisée en ce qu'**elle comprend un support acceptable d'un point de vue pharmaceutique permettant son administration à l'homme ou l'animal.

30. Cellule transfectée par un complexe selon l'une des revendications 1 à 19 ou une composition pharmaceutique selon l'une des revendications 26 à 29.

31. Polymère cationique de formule : 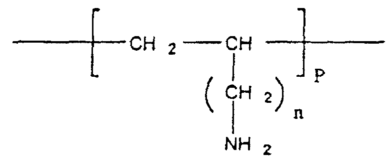 pour lequel n est un nombre entier variant de 0 à 5 et p est un nombre entier variant de 2 à 20000, plus particulièrement p varie de 10 à 18000, et avantageusement de 200 à 1000,
**caractérisé en ce que** :
- au moins 10%, avantageusement de 30 à 80%, préférentiellement 70%, des fonctions NH₂ libres sont substituées par des groupes R hydrophiles identiques ou différents, choisis parmi le polymethyloxazoline, le polyethyloxazoline, l'acide polyglycolique et les dérivés de cellulose tels que l'hydroxymethylcellulose ou l'hydroxyethylcellulose.
- ledit polymère cationique peut en outre comprendre au moins un élément de ciblage associé de manière covalente ou non aux fonctions NH₂ libres et/ou auxdits groupes R hydrophiles sous réserve que ledit polymère cationique contienne au moins 20%, préférentiellement au moins 30%, de fonctions NH₂ libres.

32. Polymère selon la revendication 31 **caractérisé en ce que** R est choisi parmi les groupes R - C = 0 et -(CH₂)_{n'}-R', où R' désigne un groupement contenant au moins une fonction hydrophile, n' est un nombre entier variant de 1 à 5.

33. Polymère selon la revendication 32 **caractérisé en ce que** R' est choisi parmi le polymethyloxazoline, le polyethyloxazoline, l'acide polyglycolique et les dérivés de cellulose tels que l'hydroxymethylcellulose ou l'hydroxyethylcellulose.

34. Polymère selon l'une des revendications 31 à 33, **caractérisé en ce qu'**il s'agit d'une polyallylamine glycolilée.

35. Polymère selon la revendication 34, **caractérisé en ce que** R est 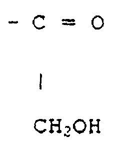 et environ 50 à environ 70 % de fonctions NH₂ libres sont substituées par R.

36. Polymère selon l'une des revendications 31 à 33, **caractérisé en ce qu'**il s'agit d'une polyallylamine éthoxylée.

37. Complexe selon la revendication 36, **caractérisé en ce que** R est (CH₂)₂-OH
et environ 50 à environ 80 % de fonctions NH₂ libres sont substituées par R.

38. Polymère selon la revendication 35 ou 37, **caractérisé en ce que** p = 592.

39. Polymère selon l'une quelconque des revendications 31 à 38 **caractérisé en ce que** ledit élément de ciblage est présent et **en ce qu'**il est choisi parmi le groupe consistant en tout ou partie de sucres, de peptides, d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogèniques, de peptides de localisation nucléaire, ou d'une combinaison de tels composés.

## Claims

1. Complex comprising at least one cationic polymer of formula 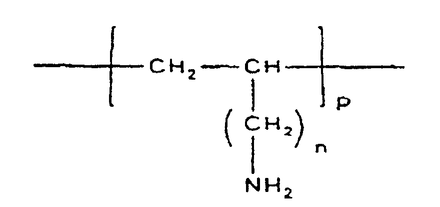 in which n is a whole number varying from 0 to 5 and p is a whole number varying from 2 to 20,000, more particularly p varies from 10 to 18,000 and advantageously from 200 to 1000,
**characterized in that**:
- at least 10%, advantageously from 30 to 80%, preferentially 70%, of the free NH₂ functions are substituted with identical or different hydrophilic R groups;
- said cationic polymer may in addition comprise at least one targeting element combined covalently or not with the free NH₂ functions and/or with said hydrophilic R groups provided that said cationic polymer contains at least 20%, preferably at least 30%, of free NH₂ functions, combined with at least one therapeutically active substance, comprising at least one negative charge.

2. Complex according to claim 1, **characterized in that** said cationic polymer corresponds to the formula: 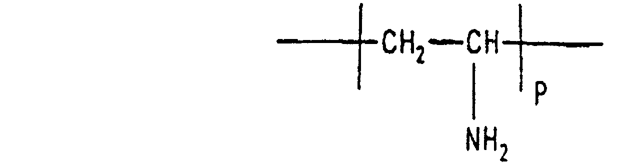 (n=0)

3. Complex according to claim 1, **characterized in that** said cationic polymer corresponds to the formula: 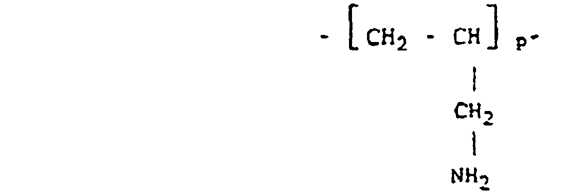 (n=1).

4. Complex according to any one of claims 1 to 3, **characterized in that** said hydrophilic R groups of said cationic polymer comprise at least one hydrophilic function chosen from the amine, hydroxyl, amide and ester functions.

5. Complex according to any one of claims 1 to 4, **characterized in that** R is chosen from the groups R'-C=O and -(CH₂)_{n'}-R' where R' designates a group containing at least one hydrophilic function n' is a whole number varying from 1 to 5.

6. Complex according to any one of claims 1 to 4, **characterized in that** R or R' consists of a polymer exhibiting hydrophilic properties.

7. Complex according to claim 6, **characterized in that** R or R' is chosen from polyethylene glycol and its derivatives, polyvinylpyrrolidone, polymethyloxazoline, polyethyloxazoline, polyhydroxypropyl methacrylamide, polylactic acid, polyglycolic acid and cellulose derivatives such as hydroxymethylcellulose or hydroxyethylcellulose.

8. Complex according to claim 7, **characterized in that** R or R' is polyethylene glycol (PEG) having a molecular weight varying from 300 to 5000, more particularly from 1000 to 4000 and advantageously having a molecular weight of 2000 (PEG 2000).

9. Complex according to either of claims 3 and 4, **characterized in that** it is a glycolilated polyallylamine.

10. Complex according to claim 9, **characterized in that** R is 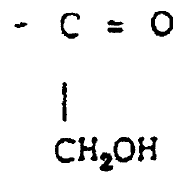 and about 50 to about 70% of free NH₂ functions are substituted with R.

11. Complex according to claim 3 or 4, **characterized in that** it is an ethoxylated polyallylamine.

12. Complex according to claim 11, **characterized in that** R is (CH₂)₂-OH and about 50 to about 80% of free NH₂ functions are substituted with R.

13. Complex according to claim 10 or 12, **characterized in that** p = 592.

14. Complex according to any one of claims 1 to 13, **characterized in that** said targeting element is present in said cationic polymer and **in that** it is chosen from the group consisting of the whole or part of sugars, peptides, oligonucleotides, lipids, hormones, vitamins, antigens, antibodies, ligands specific for membrane receptors, ligands capable of reacting with an antiligand, fusogenic peptides, nuclear localization peptides, or a combination of such compounds.

15. Complex according to claims 1 to 14, **characterized in that** the therapeutically active substance is chosen from nucleic acids and proteins.

16. Complex according to claim 15, **characterized in that** said therapeutically active substance is a nucleic acid chosen from the group consisting of a cDNA, a genomic DNA, a plasmid DNA, a messenger RNA, an antisense RNA, a ribozyme, a transfer RNA, a ribosomal RNA, or a DNA encoding such RNAs.

17. Complex according to claim 16, **characterized in that** said nucleic acid comprises a gene of interest and elements for expressing said gene of interest.

18. Complex according to claim 17, **characterized in that** said gene of interest encodes the whole or part of a ribozyme, an antisense nucleic acid, a polypeptide, in particular which is therapeutically active or is a marker.

19. Complex according to one of claims 1 to 18, **characterized in that** the ratio between the number of positive charges of said cationic polymer and the number of negative charges of said therapeutically active substance varies from 1 to 30, preferably from 1.5 to 10 and advantageously from 2.5 to 5.

20. Method of preparing a complex according to one of claims 1 to 19, **characterized in that** one or more cationic polymers of formula: 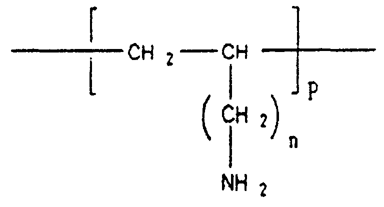 in which n is a whole number varying from 0 to 5 and p is a whole number varying from 2 to 20,000, more particularly p varies from 10 to 18,000 and advantageously from 200 to 1000, **characterized in that**:
- at least 10%, advantageously from 30 to 80%, preferentially 70%, of the free NH₂ functions are substituted with identical or different hydrophilic R groups;
- said cationic polymer may in addition comprise at least one targeting element combined covalently or not with the free NH₂ functions and/or with said hydrophilic R groups provided that said cationic polymer contains at least 20%, preferably at least 30%, of free NH₂ functions,
with one or more therapeutically active substances comprising at least one negative charge and **in that** said complex is recovered optionally after a purification step.

21. Method for transferring a therapeutically active substance into a target cell in vitro, **characterized in that** target cells are brought into contact with at least one complex according to any one of claims 1 to 19.

22. Method according to claim 21, **characterized in that** said target cell is chosen from prokaryotic cells, yeast cells, eukaryotic cells, in particular animal cells, and more particularly mammalian cells, especially human and/or cancer cells.

23. Use of a complex according to one of claims 1 to 19 for the preparation of a medicament for the treatment of the human or animal body, in particular by gene therapy.

24. Use according to claim 23, **characterized in that** the medicament is intended to be administered in an injectable form, especially by the intramuscular route.

25. Use according to claim 23, **characterized in that** the medicament is intended to be administered by inhalation, by instillation, by aerosolization, by the topical route or by the oral route.

26. Pharmaceutical composition, **characterized in that** it comprises at least one complex according to any one of claims 1 to 19.

27. Composition according to claim 26, **characterized in that** it comprises at least one adjuvant capable of enhancing the capacity for transfection of said complex into a target cell in vitro, ex vivo or in vivo.

28. Composition according to claim 27, **characterized in that** said adjuvant is chosen from the group consisting of a lysosomotropic agent such as for example chloroquine, a protic polar compound chosen in particular from propylene glycol, polyethylene glycol, glycerol, ethanol, 1-methyl-L-2-pyrrolidone or derivatives thereof, and an aprotic polar compound chosen in particular from dimethyl sulfoxide (DMSO), diethyl sulfoxide, di-n-propyl sulfoxide, dimethyl sulfone, sulfolane, dimethylformamide, dimethylacetamide, tetramethylurea, acetonitrile or derivatives thereof.

29. Composition according to one of claims 26 to 28, **characterized in that** it comprises a pharmaceutically acceptable carrier allowing its administration to humans or animals.

30. Cell transfected with a complex according to one of claims 1 to 19 or a pharmaceutical composition according to one of claims 26 to 29.

31. Cationic polymer of formula I: 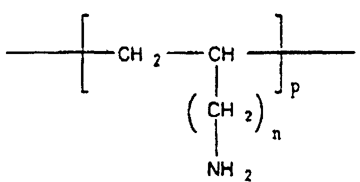 in which n is a whole number varying from 0 to 5 and p is a whole number varying from 2 to 20,000, more particularly p varies from 10 to 18,000 and advantageously from 200 to 1000,
**characterized in that**:
- at least 10%, advantageously from 30 to 80%, preferentially 70%, of the free NH₂ functions are substituted with identical or different hydrophilic R groups, chosen from polymethyloxazoline, polyethyloxazoline, polyglycolic acid and cellulose derivatives such as hydroxymethylcellulose or hydroxyethylcellulose,
- said cationic polymer may in addition comprise at least one targeting element combined covalently or not with the free NH₂ functions and/or with said hydrophilic R groups provided that said cationic polymer contains at least 20%, preferably at least 30%, of free NH₂ functions.

32. Polymer according to claim 31, **characterized in that** R is chosen from the groups R-C=O and -(CH₂)_{n'}-R' where R' designates a group containing at least one hydrophilic function, n' is a whole number varying from 1 to 5.

33. Polymer according to claim 32, **characterized in that** R' is chosen from polymethyloxazoline, polyethyloxazoline, polyglycolic acid and cellulose derivatives such as hydroxymethylcellulose or hydroxyethylcellulose.

34. Polymer according to one of claims 31 to 33, **characterized in that** it is a glycolilated polyallylamine.

35. Polymer according to claim 34, **characterized in that** R is 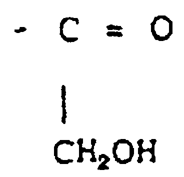 and about 50 to about 70% of free NH₂ functions are substituted with R.

36. Polymer according to one of claims 31 to 33, **characterized in that** it is an ethoxylated polyallylamine.

37. Polymer according to claim 36, **characterized in that** R is (CH₂)₂-OH
and about 50 to about 80% of free NH₂ functions are substituted with R.

38. Polymer according to claim 35 or 37, **characterized in that** p = 592.

39. Polymer according to any one of claims 31 to 38, **characterized in that** said targeting element is present and **in that** it is chosen from the group consisting of the whole or part of sugars, peptides, oligonucleotides, lipids, hormones, vitamins, antigens, antibodies, ligands specific for membrane receptors, ligands capable of reacting with an antiligand, fusogenic peptides, nuclear localization peptides, or a combination of such compounds.

## Patentansprüche

1. Komplex, umfassend mindestens ein kationisches Polymer der Formel: 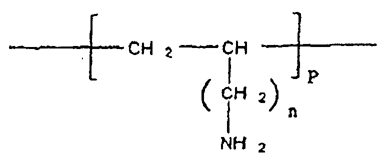 in dem n eine ganze Zahl von 0 bis 5 ist und p eine ganze Zahl von 2 bis 20000 ist, p spezieller von 10 bis 18000 und vorteilhaft von 200 bis 1000 variiert,
**dadurch gekennzeichnet, dass**:
- mindestens 10%, vorteilhaft 30 bis 80%, bevorzugt 70% der freien NH₂-Funktionen durch identische oder verschiedene hydrophile Gruppen R substituiert sind,
- das kationische Polymer darüber hinaus mindestens ein Targetelement umfassen kann, das auf kovalente oder nicht-kovalente Weise mit den freien NH₂-Funktionen und/oder den hydrophilen Gruppen R verknüpft ist, mit der Maßgabe, dass das kationische Polymer mindestens 20%, vorzugsweise mindestens 30%, freie NH₂-Funktionen enthält,
verbunden mit mindestens einer therapeutisch aktiven Substanz, die mindestens eine negative Ladung enthält.

2. Komplex nach Anspruch 1, **dadurch gekennzeichnet, dass** das kationische Polymer der Formel: 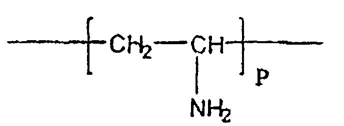 entspricht (n = 0).

3. Komplex nach Anspruch 1, **dadurch gekennzeichnet, dass** das kationische Polymer der Formel: 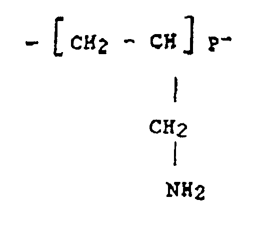 entspricht (n = 1).

4. Komplex nach irgendeinem der Ansprüche 1 bis 3, **dadurch gekennzeichnet, dass** die hydrophilen Gruppen R des kationischen Polymers mindestens eine hydrophile Funktion umfassen, die ausgewählt ist aus den Funktionen Amin, Hydroxyl, Amid und Ester.

5. Komplex nach irgendeinem der Ansprüche 1 bis 4, **dadurch gekennzeichnet, dass** R ausgewählt ist aus den Gruppen R'-C=O und -(CH₂)_{n'}-R', worin R' eine Gruppierung bezeichnet, die mindestens eine hydrophile Funktion enthält, n' eine ganze Zahl von 1 bis 5 ist.

6. Komplex nach irgendeinem der Ansprüche 1 bis 4, **dadurch gekennzeichnet, dass** R oder R' aus einem Polymer besteht, das hydrophile Eigenschaften aufweist.

7. Komplex nach Anspruch 6, **dadurch gekennzeichnet, dass** R oder R' ausgewählt ist aus Polyethylenglycol und seinen Derivaten, Polyvinylpyrrolidon, Polymethyloxazolin, Polyethyloxazolin, Polyhydroxypropylmethacrylamid, Polymilchsäure, Polyglycolsäure und Cellulose-Derivaten, wie Hydroxymethylcellulose oder Hydroxyethylcellulose.

8. Komplex nach Anspruch 7, **dadurch gekennzeichnet, dass** R oder R' Polyethylenglycol (PEG) ist, das ein Molekulargewicht von 300 bis 5000, spezieller von 1000 bis 4000 aufweist und vorteilhaft ein Molekulargewicht von 2000 (PEG 2000) aufweist.

9. Komplex nach einem der Ansprüche 3 oder 4, **dadurch gekennzeichnet, dass** es sich um ein glycolisiertes Polyallylamin handelt.

10. Komplex nach Anspruch 9, **dadurch gekennzeichnet, dass** R für 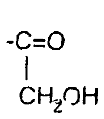 steht und etwa 50 bis etwa 70% der freien NH₂-Funktionen durch R substituiert sind.

11. Komplex nach Anspruch 3 oder 4, **dadurch gekennzeichnet, dass** es sich um ein ethoxyliertes Polylallylamin handelt.

12. Komplex nach Anspruch 11, **dadurch gekennzeichnet, dass** R für (CH₂)₂-OH steht und etwa 50 bis etwa 80% der freien NH₂-Funktionen durch R substituiert sind.

13. Komplex nach Anspruch 10 oder 12, **dadurch gekennzeichnet, dass** p = 592.

14. Komplex nach irgendeinem der Ansprüche 1 bis 13, **dadurch gekennzeichnet, dass** das Targetelement in dem kationischen Polymer anwesend ist und dass es ausgewählt ist aus der Gruppe bestehend aus ganzen oder einem Teil von Zuckern, Peptiden, Oligonukleotiden, Lipiden, Hormonen, Vitaminen, Antigenen, Antikörpern, spezifischen Liganden von Membranrezeptoren, Liganden, die mit einem Antiliganden reagieren können, Fusionspeptiden, Zellkern-Peptiden oder einer Kombination derartiger Verbindungen.

15. Komplex nach irgendeinem der Ansprüche 1 bis 14, **dadurch gekennzeichnet, dass** die therapeutisch aktive Substanz ausgewählt ist aus Nukleinsäuren und Proteinen.

16. Komplex nach Anspruch 15, **dadurch gekennzeichnet, dass** die therapeutisch aktive Substanz eine Nukleinsäure ist, die ausgewählt ist aus der Gruppe bestehend aus einer cDNS, einer genomischen DNS, einer Plasmid-DNS, einer Boten-RNS, einer Antisinn-RNS, einem Ribozym, einer Transfer-RNS, einer ribosomischen RNS oder einer DNS, die für derartige RNS kodiert.

17. Komplex nach Anspruch 16, **dadurch gekennzeichnet, dass** die Nukleinsäure ein interessierendes Gen und Elemente zur Expression des interessierenden Gens umfasst.

18. Komplex nach Anspruch 17, **dadurch gekennzeichnet, dass** das interessierende Gen für das Ganze oder einen Teil eines Ribozyms, einer Antisinn-Nukleinsäure, eines Polypeptids, insbesondere eines therapeutisch aktiven oder Marker-Polypeptids, kodiert.

19. Komplex nach einem der Ansprüche 1 bis 18, **dadurch gekennzeichnet, dass** das Verhältnis zwischen der Zahl der positiven Ladungen des kationischen Polymers und der Zahl der negativen Ladungen der therapeutisch aktiven Substanz von 1 bis 30, vorzugsweise 1,5 bis 10 und vorteilhaft von 2,5 bis 5 variiert.

20. Verfahren zur Herstellung eines Komplexes nach irgendeinem der Ansprüche 1 bis 19, **dadurch gekennzeichnet, dass** man ein oder mehrere kationische Polymere der Formel: 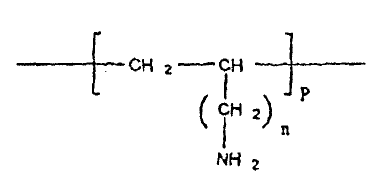 worin n eine ganze Zahl von 0 bis 5 ist und p eine ganze Zahl von 2 bis 20000 ist, spezieller p von 10 bis 18000 und vorteilhaft von 200 bis 1000 variiert, **dadurch gekennzeichnet, dass**
- mindestens 10%, vorteilhaft 30 bis 80%, bevorzugt 70% der freien NH₂-Funktionen durch identische oder verschiedene hydrophile Gruppen R substituiert sind,
- das kationische Polymer darüber hinaus mindestens ein Targetelement umfassen kann, das auf kovalente oder nicht-kovalente Weise mit den freien NH₂-Funktionen und/oder den hydrophilen Gruppen R verknüpft ist, mit der Maßgabe, dass das kationische Polymer mindestens 20%, vorzugsweise mindestens 30%, freie NH₂-Funktionen enthält,
mit einer oder mehreren therapeutisch aktiven Substanzen in Kontakt bringt, welche mindestens eine negative Ladung umfassen, und dass man den Komplex gegebenenfalls nach einem Reinigungsschritt gewinnt.

21. Verfahren zum Überführen einer therapeutisch aktiven Substanz in eine Targetzelle in vitro, **dadurch gekennzeichnet, dass** man die Targetzellen mit mindestens einem Komplex nach irgendeinem der Ansprüche 1 bis 19 in Kontakt bringt.

22. Verfahren nach Anspruch 21, **dadurch gekennzeichnet, dass** die Targetzelle ausgewählt ist aus prokaryontischen Zellen, Hefezellen, eukaryontischen Zellen, insbesondere Tierzellen und spezieller Säugerzellen, insbesondere menschlichen und/oder Krebs-Zellen.

23. Verwendung eines Komplexes nach irgendeinem der Ansprüche 1 bis 19 für die Herstellung eines Medikaments für die Behandlung des menschlichen oder tierischen Körpers, insbesondere durch Gentherapie.

24. Verwendung nach Anspruch 23, **dadurch gekennzeichnet, dass** das Medikament dazu bestimmt ist, in injizierbarer Form, insbesondere auf intramuskularem Weg, verabreicht zu werden.

25. Verwendung nach Anspruch 23, **dadurch gekennzeichnet, dass** das Medikament dazu bestimmt ist, durch Inhalation, durch Instillation, durch Aerolisierung, auf topischem Weg oder auf oralem Weg verabreicht zu werden.

26. Pharmazeutische Zusammensetzung, **dadurch gekennzeichnet, dass** sie mindestens einen Komplex nach irgendeinem der Ansprüche 1 bis 19 umfasst.

27. Zusammensetzung nach Anspruch 26, **dadurch gekennzeichnet, dass** sie mindestens ein Adjuvans umfasst, das in der Lage ist, das Vermögen der Transfektion des Komplexes in das Innere einer Targetzelle in vitro, ex vivo oder in vivo zu verbessern.

28. Zusammensetzung nach Anspruch 27, **dadurch gekennzeichnet, dass** das Adjuvans ausgewählt ist aus der Gruppe bestehend aus einem lysosomotropen Mittel, wie Chloroquin, einer polaren protischen Verbindung, ausgewählt aus Propylenglycol, Polyethylenglycol, Glycerin, Ethanol, 1-Methyl-L-2-pyrrolidon oder deren Derivaten, und einer aprotischen polaren Verbindung, insbesondere ausgewählt aus Dimethylsulfoxid (DMSO), Diethylsulfoxid, Di-n-propylsulfoxid, Dimethylsulfon, Sulfolan, Dimethylformamid, Dimethylacetamid, Tetramethylharnstoff, Acetonitril oder deren Derivaten.

29. Zusammensetzung nach einem der Ansprüche 26 bis 28, **dadurch gekennzeichnet, dass** sie einen Träger umfasst, der unter einem pharmazeutischen Gesichtspunkt annehmbar ist und ihre Verabreichung beim Menschen oder beim Tier gestattet.

30. Zelle, die mit einem Komplex nach irgendeinem der Ansprüche 1 bis 19 oder einer pharmazeutischen Zusammensetzung nach einem der Ansprüche 26 bis 29 transfiziert ist.

31. Kationisches Polymer der Formel: 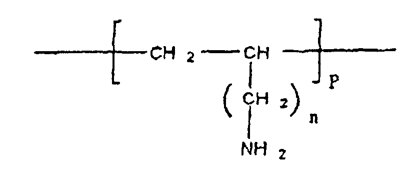 in dem n eine ganze Zahl von 0 bis 5 ist und p eine ganze Zahl von 2 bis 20000 ist, p spezieller von 10 bis 18000 und vorteilhaft von 200 bis 1000 variiert,
**dadurch gekennzeichnet, dass**;
- mindestens 10%, vorteilhaft 30 bis 80%, bevorzugt 70% der freien NH₂-Funktionen durch identische oder verschiedene hydrophile Gruppen R substituiert sind, die ausgewählt sind aus Polymethyloxazolin, Polyethyloxazolin, Polyglycolsäure und Cellulose-Derivaten, wie Hydroxymethylcellulose oder Hydroxyethylcellulose,
- das kationische Polymer darüber hinaus mindestens ein Targetelement umfassen kann, das auf kovalente oder nicht-kovalente Weise mit den freien NH₂-Funktionen und/oder den hydrophilen Gruppen R verknüpft ist, mit der Maßgabe, dass das kationische Polymer mindestens 20%, vorzugsweise mindestens 30%, freie NH₂-Funktionen enthält.

32. Polymer nach Anspruch 31, **dadurch gekennzeichnet, dass** R ausgewählt ist aus den Gruppen R-C=O und -(CH₂)_{n'}-R', worin R' eine Gruppierung bezeichnet, die mindestens eine hydrophile Funktion enthält, n' eine ganze Zahl von 1 bis 5 ist.

33. Polymer nach Anspruch 32, **dadurch gekennzeichnet, dass** R' ausgewählt ist aus Polymethyloxazolin, Polyethyloxazolin, Polyglycolsäure und Cellulose-Derivaten, wie Hydroxymethylcellulose oder Hydroxyethylcellulose.

34. Polymer nach einem der Ansprüche 31 bis 33, **dadurch gekennzeichnet, dass** es sich um glycolisiertes Polyallylamin handelt.

35. Polymer nach Anspruch 34, **dadurch gekennzeichnet, dass** R für 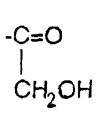 steht und etwa 50 bis etwa 70% der freien NH₂-Funktionen durch R substituiert sind.

36. Polymer nach einem der Ansprüche 31 bis 33, **dadurch gekennzeichnet, dass** es sich um ein ethoxyliertes Polyallylamin handelt.

37. Komplex nach Anspruch 36, **dadurch gekennzeichnet, dass** R für (CH₂)₂-OH steht und etwa 50 bis etwa 80% der freien NH₂-Funktionen durch R substituiert sind.

38. Polymer nach Anspruch 35 oder 37, **dadurch gekennzeichnet, dass** p = 592.

39. Polymer nach irgendeinem der Ansprüche 31 bis 38, **dadurch gekennzeichnet, dass** das Targetelement anwesend ist und dadurch, dass es ausgewählt ist aus der Gruppe bestehend aus ganzen oder einem Teil von Zuckern, Peptiden, Oligonukleotiden, Lipiden, Hormonen, Vitaminen, Antigenen, Antikorpern, spezifischen Liganden von Membranrezeptoren, Liganden, die mit einem Antiliganden reagieren können, Fusionspeptiden, Zellkern-Peptiden oder einer Kombination derartiger Verbindungen.
